# EUROPÄISCHE PATENTSCHRIFT

(11) **EP 2 812 439 B1**
(45) Veröffentlichungstag und Bekanntmachung des Hinweises auf die Patenterteilung: **22.05.2019**
(21) Anmeldenummer: 13703568.9
(22) Anmeldetag: 06.02.2013
(51) Int. Cl.: C12P 19/02, C12P 19/36, C12P 33/00, C12P 41/00, C13K 11/00

(54) **VERFAHREN ZUR ENZYMATISCHEN REGENERIERUNG VON REDOXKOFAKTOREN**
METHOD FOR ENZYMATIC REGENERATION OF REDOX COFACTORS
PROCÉDÉ DESTINÉ À LA RÉGÉNÉRATION ENZYMATIQUE DE CO-FACTEURS REDOX

(30) Priorität: 07.02.2012 EP 12450007; 12.09.2012 WO PCT/EP2012/067781; 10.12.2012 AT 12842012
(43) Veröffentlichungstag der Anmeldung: 17.12.2014
(73) Patentinhaber: Annikki GmbH, 8074 Raaba-Grambach (AT)
(72) Erfinder: ERTL, Ortwin, 8076 Vasoldsberg (AT); STAUNIG, Nicole, 8076 Vasoldsberg (AT); SUT-VEJDA, Marta, A-8010 Graz (AT); MAYER, Bernd, D-88453 Erolzheim (DE)
(74) Vertreter: Schwarz & Partner Patentanwälte OG
(86) Internationale Anmeldenummer: PCT/EP2013/052313
(87) Internationale Veröffentlichungsnummer: WO 2013/117584

(56) Entgegenhaltungen:
- WO-A1-86/04353
- WO-A1-2007/118644
- DANIELA MONTI ET AL: "One-pot multienzymatic synthesis of 12-ketoursodeoxycholic acid: subtle cofactor specificities rule the reaction equilibria of five biocatalysts working in a row", ADVANCED SYNTHESIS & CATALYSIS, WILEY VCH VERLAG, WEINHEIM, DE, Bd. 351, Nr. 9, 1. Juni 2009 (2009-06-01), Seiten 1303-1311, XP002666468, ISSN: 1615-4150, DOI: 10.1002/ADSC.200800727 [gefunden am 2009-03-17] in der Anmeldung erwähnt
- JOERG H SCHRITTWIESER ET AL: "Recent biocatalytic oxidationreduction cascades", CURRENT OPINION IN CHEMICAL BIOLOGY, Bd. 15, Nr. 2, April 2011 (2011-04), Seiten 249-256, XP028187359, ISSN: 1367-5931, DOI: 10.1016/J.CBPA.2010.11.010 [gefunden am 2010-11-11]
- CONSTANCE V. VOSS ET AL: "Orchestration of Concurrent Oxidation and Reduction Cycles for Stereoinversion and Deracemisation of sec -Alcohols", JOURNAL OF THE AMERICAN CHEMICAL SOCIETY, Bd. 130, Nr. 42, 22. Oktober 2008 (2008-10-22), Seiten 13969-13972, XP055033012, ISSN: 0002-7863, DOI: 10.1021/ja804816a
- SHIN-ICHIRO SUYE ET AL: "Enzymatic production of l-alanine from malic acid with malic enzyme and alanine dehydrogenase with coenzyme regeneration", THE CANADIAN JOURNAL OF CHEMICAL ENGINEERING, Bd. 70, Nr. 2, 1. April 1992 (1992-04-01), Seiten 306-312, XP055033010, ISSN: 0008-4034, DOI: 10.1002/cjce.5450700214 in der Anmeldung erwähnt
- RYAN WOODYER ET AL: "Mechanistic investigation of a highly active phosphite dehydrogenase mutant and its application for NADPH regeneration", FEBS JOURNAL, Bd. 272, Nr. 15, 1. August 2005 (2005-08-01), Seiten 3816-3827, XP055033097, ISSN: 1742-464X, DOI: 10.1111/j.1742-4658.2005.04788.x in der Anmeldung erwähnt

## Beschreibung

### Hintergrund der Erfindung

Die vorliegende Erfindung betrifft ein Verfahren zur enzymatischen Regenerierung der Redoxkofaktoren NAD⁺/NADH und NADP⁺/NADPH in einer Ein-Topf-Reaktion, wobei als Resultat mindestens zweier weiterer im selben Reaktionsansatz ablaufender enzymatisch katalysierter Redoxreaktionen (= Produktbildungsreaktionen) einer der beiden Redoxkofaktoren in seiner reduzierten Form anfällt und der jeweils andere in seiner oxidierten Form.

### Stand der Technik

Enzymkatalysierte Redoxreaktionen werden in industriellen Prozessen beispielsweise in der Herstellung von chiralen Alkoholen, α-Aminosäuren und α-Hydroxysäuren verwendet. Die Mehrheit der Enzyme, die in industriellen Redoxreaktionen zum Einsatz kommen, verwendet Kofaktoren wie NADH oder NADPH. Unter den enzymatischen Redoxreaktionen sind jene besonders interessant, bei denen die Redox-Kofaktoren durch *in situ* Kofaktor-Regenerierungssysteme wiederhergestellt werden. Der Grund dafür liegt darin, dass die Verwendung von nur katalytischen Mengen der teuren Kofaktoren (NAD(P)⁺/NAD(P)H) möglich ist. Die Erreichbarkeit von geeigneten Dehydrogenasen und anderen Enzymen hat zur Entwicklung diverser Kofaktor-Regenerierungssysteme geführt.

Die bis jetzt beschriebenen Regenerationssysteme könnte man klassifizieren als: enzymgekoppelt, substratgekoppelt, *in vivo* (natürliche Kofaktor-Regenerierungssysteme in lebenden Organismen), photochemisch, chemisch oder elektro-enzymatisch. Das hier beschriebene Verfahren betrifft ein enzymgekoppeltes Regenerierungssystem. Vorteile von enzymgekoppelten Systemen sind die hohe Selektivität, die Anwendbarkeit zur Herstellung verschiedener Produkte und die hohe Wiederverwendungsrate des Kofaktors (*total turnover number*, TTN).

Mitte der 1990er Jahre wurde ein erster industrieller Prozess unter Verwendung eines enzymgekoppelten Kofaktor-Regenerierungssystems im Tonnen-Maßstab angewendet. In diesem Prozess wurde Formatdehydrogenase aus *Candida boidinii* eingesetzt. Die bisher bekannten industriellen Prozesse verwenden in der Regel ein Redoxenzym zur Produktsynthese sowie ein weiteres Enzym zur Kofaktor-Regenerierung.

Davon zu unterscheiden sind Verfahren, bei denen zwei oder mehr an der Produktbildung beteiligte enzymatische Redoxreaktionen und zwei enzymatische Systeme zur Kofaktor-Regenerierung (zeitgleich oder sequentiell) in einem Reaktionsansatz ablaufen, ohne dass ein Zwischenprodukt isoliert wird. In letzter Zeit haben solche enzymatischen Kaskadenreaktionen - hier als Ein-Topf-Reaktionen bezeichnet - signifikante Aufmerksamkeit erregt da sie effektiv Betriebskosten, Betriebszeit und Umweltauswirkungen reduzieren. Zusätzlich ermöglichen enzymatische Kaskaden von Redoxreaktionen Transformationen, die durch klassische chemische Verfahren nicht einfach umzusetzen sind.

Es ist allerdings eine Herausforderung, mehrere Reaktionen (Oxidation und Reduktion) in einer Ein-Topf-Reaktion mit paralleler Kofaktor-Regenerierung gleichzeitig durchzuführen, da oft sehr divergente Reaktionsbedingungen für die einzelnen Transformationen notwendig sind. Bis jetzt wurden nur sehr wenige Ein-Topf-Versuche umfassend Oxidations- und Reduktionsreaktionen mit zugehörigen Kofaktor-Regenerierungssystemen durchgeführt.

In der Literatur (Advanced Synth. Catal., 2009, Volume 351, Issue 9, p1303-1311) wurde der Versuch einer Ein-Topf-Reaktion unter Verwendung von 7α-Hydroxysteroiddehydrogenase (HSDH), 7β-HSDH und 12α-HSDH beschrieben. In dem Verfahren wurde eine sowohl regioselektive als auch stereoselektive Oxidation an den Positionen 7 und 12 von Cholsäure durchgeführt, gefolgt von einer regio- und stereoselektiven Reduktion an Position 7. In dem Prozess wurde als Kofaktor-Regenerierungssystem sowohl eine Laktatdehydrogenase (NAD⁺-abhängig) als auch eine Glukosedehydrogenase (NADP⁺-abhängig) verwendet. Als Kosubstrate wurden Pyruvat und Glukose verwendet. Obwohl dieses Verfahren ursprünglich auf einen echten Ein-Topf-Prozess abzielte, wurden letztendlich Oxidations- und Reduktionsreaktion getrennt ausgeführt. Dabei erfolgte die Aufteilung von oxidativen und reduktiven Schritten entweder in einem sogenannten "Teebeutel"-Reaktor oder im Membranreaktor. Diese Aufteilung war notwendig, um aufgrund der niedrigen Kofaktor-Selektivität von NADPH-Glukosedehydrogenase die Produktion von Nebenprodukten zu vermeiden. In der Ein-Topf-Reaktion setzte die Glukosedehydrogenase NADP⁺ aber teilweise auch NAD⁺ um, was die Oxidation behinderte. In dem beschriebenen Prozess wurden nur 12,5 mM (∼0,5%) des Substrats Cholsäure eingesetzt, was den Prozess ökonomisch uninteressant macht.

Es wurde weiterhin ein Versuch beschrieben, die Deracemisierung von Racematen sekundärer Alkohole über ein prochirales Keton als Zwischenprodukt unter Verwendung eines Ein-Topf-Systems durchzuführen (J. Am. Chem. Soc., 2008, Volume 130, p13969-13972). Die Deracemisierung von sekundären Alkoholen wurde über zwei Alkoholdehydrogenasen (S- und R-spezifisch) mit unterschiedlicher Kofaktor-Spezifizität erreicht. In dem System wurde NADP durch NADPH Oxydase (Wasserestoffperoxyd produzierende) und NADH durch Formiatdehydrogenase regeneriert. Als Kosubstrate wurden Formiat und Sauerstoff verwendet. In dem System wurden 4 Enzyme ohne Aufteilung von oxidativen und reduktiven Schritten eingesetzt. Ein Nachteil des Verfahren ist die sehr geringe Konzentration des eingesetzten Substrats von 0,2-0,5%, was für industrielle Zwecke nicht geeignet ist.

Ein weiteres Ein-Topf-System wurde in WO 2009/121785 A2 beschrieben. In dem Verfahren wurde ein Stereoisomer eines optisch aktiven sekundären Alkohols zum Keton oxidiert und dann zum entsprechenden optischen Antipoden reduziert, wobei zwei Alkoholdehydrogenasen mit entgegengesetzten Stereoselektivitäten und unterschiedlichen Kofaktor-Spezifitäten verwendet wurden. Die Kofaktoren wurde mittels eines sogenannten "Hydrid-Transfer-Systems" unter Verwendung nur eines zusätzlichen Enzyms regeneriert. Um die Kofaktoren zu regenerieren wurden verschiedene Enzyme wie z.B. Formiatdehydrogenase, Glukosedehydrogenase, Lactatdehydrogenase verwendet. Ein Nachteil dieses Verfahrens ist die geringe Konzentration der verwendeten Substrate.

Der Nachteil der bis jetzt bekannten enzymatischen Ein-Topf-Verfahren mit Kofaktor-Regenerierungssystemen ist insgesamt die sehr geringe Substratkonzentration, was für industrielle Prozesse unwirtschaftlich ist.

Im Gegensatz dazu sind bereits viele einzelne enzymatische Redoxreaktionen bekannt, bei denen Kofaktor-Regenerierungssysteme verwendet werden. Die Versuche wurden mit ganzen Mikroorganismen, Zell-Lysaten oder isolierten Enzymen mit gleichzeitiger NAD(P)H- oder NAD(P)⁺-Regenerierung beschrieben. Bekannte enzymatische Kofaktor-Regenerierungssysteme für einzelne Redoxreaktionen beinhalten beispielsweise Formiatdehydrogenase für NADH (Formiat als Kosubstrat), Alkoholdehydrogenase aus *Pseudomonas sp.* für NADH (2-Propanol als Kosubstrat), Hydrogenase für NADH und NADPH (H₂ als Kosubstrat), Glukose-6-phosphatdehydrogenase aus *L. mesenteroides* für NADPH (Glukose-6-phosphat als Kosubstrat), Glukosedehydrogenase für NADH und NADPH (Glukose als Kosubstrat), NADH Oxydase für NADH (O₂ als Kosubstrat) und Phosphitdehydrogenase für NADH (Phosphit als Kosubstrat).

Ein Anwendungsbeispiel solcher einzelner Redoxreaktionen ist die Herstellung chiraler Hydroxyverbindungen ausgehend von entsprechenden prochiralen Ketoverbindungen. In diesen Verfahren wird der Kofaktor mittels eines zusätzlichen Enzyms regeneriert. Diesen Verfahren ist gemeinsam, dass sie eine isolierte Reduktionreaktion darstellen und NAD(P)H regenerieren (siehe z.B. EP 1 152 054).

Enzymatische Verfahren unter Verwendung von Hydroxysteroiddehydrogenasen gekoppelt mit Kofaktor-Regenerierungssystem, die bei höheren Substratkonzentrationen (ca. >1%) ablaufen wurden beschrieben (EP 1 731 618; WO 2007/118644; Appl. Microbiol. Biotechnol., 2011 Volume 90 p127-135). In den Verfahren wurden die Kofaktoren NAD(P)H oder NAD(P) mittels verschiedene Enzyme wie zum Beispiel Laktatdehydrogenase (Pyruvat als Kosubstrat), Alkoholdehydrogenase aus *T*. *brockii* (Isopropanol als Kosubstrat), Alkoholdehydrogenase aus *L. brevis*, *L. minor, Leuconostoc carnosum, T. ethanolicus, Clostridium beijerinckii* regeneriert. Diese bekannten Verfahren beziehen sich jedoch lediglich auf die isolierten Einzelreaktionen zur Oxidation von Hydroxyverbindung oder zur Reduktion von Oxoverbindung.

Ein Kofaktor-Regenerierungssystem für NADH unter Verwendung von Malatdehydrogenase ("Malatenzym") wurde bereits beschrieben (Can. J. Chem. Eng. 1992, Volume 70, p 306-312). In der Publikation wurde es zur reduktiven Aminierung von Pyruvat durch Alanindehydrogenase verwendet. Das bei der Kofaktor-Regenerierung entstehende Pyruvat wurde anschließend in der produktbildenden Reaktion eingesetzt.

In der WO 2004/022764 ist ebenfalls beschrieben, NADH durch Malatdehydrogenase zu regenerieren. Anders als in der vorher beschriebenen Publikation wurde das bei der oxidativen Decarboxylierung von Malat entstehende Pyruvat nicht weiterverwendet.

Ein Beispiel einer enzymatischen Reduktion von D-Xylose zu Xylitol mit Kofaktor-Regenerierungssystem wurde beschrieben (FEBS J., 2005, Volume 272, p 3816- 3827). Als Kofaktor-Regenerierungsenzym wurde eine NADPH-abhängige Mutante von Phosphitdehydrogenase aus *Pseudomonas sp.* verwendet. Auch hierbei handelt es sich um eine Einzelreaktion zur Produktbildung.

Weitere Beispiele einer enzymatischen Herstellung von chiralen enantiomerenangereicherten organischen Verbindungen, wie zum Beispiel Alkoholen oder Aminosäuren wurden beschrieben (Organic Letters, 2003, Volume 5, p. 3649-3650; US 7,163,815; Biochem. Eng. J., 2008, Volume 39(2) p. 319-327; EP 1285 962). In den Systemen wurde als Kofakor-Regenerierungsenzym eine NAD(P)H-abhängige Oxidase aus *Lactobacillus brevis* oder *Lactobacillus sanfranciscensis* verwendet. Bei den Versuchen handelt es sich auch um Einzelreaktionen zur Produktbildung.

In WO 2011/000693 wird eine 17beta-Hydroxysteroiddehydrogenase sowie ein Verfahren beschrieben, mit dem es möglich ist, Redoxreaktionen an Position 17 von 4-Androsten-3,17-dion durchzuführen. Hierbei handelt es sich wiederum um eine isolierte Reduktionsreaktion. Es entfallen bei den genannten einzeln ablaufenden Oxidations- oder Reduktionsreaktionen die Vorteile einer Ein-Topf-Reaktion, wie z.B. Wirtschaftlichkeit durch Zeit- und Materialersparnis sowie besserer Umsatz durch enzymatische Kaskadenreaktionen.

### Aufgabenstellung und Beschreibung des Verfahrens

Ziel der vorliegenden Erfindung war die Bereitstellung eines Verfahrens zur Regenerierung der Redox-Kofaktoren NAD⁺/NADH und/oder, z.B. und, NADP⁺/NADPH, um damit zwei oder mehr enzymatisch katalysierte Redoxreaktionen in einem Reaktionsansatz wirtschaftlich durchzuführen.

Diese Aufgabe wird gemäß vorliegender Erfindung in einem Verfahren der eingangs genannten Art dadurch gelöst, dass ein Verfahren zur enzymatischen Regenerierung der Redoxkofaktoren NAD⁺/NADH und NADP⁺/NADPH in einer Ein-Topf-Reaktion bereitgestellt wird, wobei als Resultat mindestens zweier weiterer im selben Reaktionsansatz ablaufender enzymatisch katalysierter Redoxreaktionen (Produktbildungsreaktionen) der Redoxkofaktor NAD⁺/NADH in seiner reduzierten Form als NADH anfällt und der Redoxkofaktor NADP⁺/NADPH in seiner oxidierten Form als NADP⁺, dadurch gekennzeichnet, dass
a) bei der Regenerierungsreaktion, die NADH wieder in NAD⁺ überführt, Sauerstoff mittels einer NADH Oxidase oder Pyruvat mittels einer Laktatdehydrogenase reduziert wird, und
b) bei der Regenerierungsreaktion, die NADP⁺ wieder in NADPH überführt, 2-Propanol mittels einer Alkoholdehydrogenase oder Malat mittels einer Malatdehydrogenase oxidiert wird.

Ein Verfahren, das gemäß vorliegender Erfindung bereitgestellt wird, wird hierin auch als "Verfahren gemäß (nach) vorliegender Erfindung" bezeichnet.

Die Anmeldung offenbart auch allgemein ein Verfahren zur enzymatischen Regenerierung der Redoxkofaktoren NAD⁺/NADH und/oder, z.B. und, NADP⁺/NADPH in einer Ein-Topf-Reaktion, wobei als Resultat mindestens zweier weiterer im selben Reaktionsansatz ablaufender enzymatisch katalysierter Redoxreaktionen (Produktbildungsreaktionen) einer der beiden Redoxkofaktoren in seiner reduzierten Form anfällt und der jeweils andere in seiner oxidierten Form,
worin
a) bei der Regenerierungsreaktion, die den reduzierten Kofaktor wieder in seine ursprüngliche oxidierte Form überführt, Sauerstoff oder eine Verbindung der allgemeinen Formel 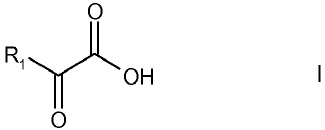 worin R₁ für eine geradkettige oder verzweigtkettige (C₁-C₄)-Alkylgruppe oder für eine (C₁-C₄)-Carboxyalkylgruppe steht, reduziert wird, und
b) bei der Regenerierungsreaktion, die den oxidierten Kofaktor wieder in seine ursprüngliche reduzierte Form überführt, ein (C₄-C₈)-Cycloalkanol oder eine Verbindung der allgemeinen Formel 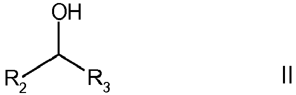 worin R₂ und R₃ unabhängig voneinander ausgewählt sind aus der Gruppe bestehend aus H, (C₁-C₆)-Alkyl, worin Alkyl geradkettig oder verzweigt ist, (C₁-C₆)-Alkenyl, worin Alkenyl geradkettig oder verzweigt ist und ein bis drei Doppelbindungen enthält, Aryl, insbesondere C₆-C₁₂ Aryl, Carboxyl, oder (C₁-C₄)-Carboxyalkyl, insbesondere auch Cycloalkyl, z.B. C₃-C₈ Cycloalkyl, oxidiert wird.

Dabei können R₂ und R₃ unabhängig ausgewählt sein aus der Gruppe, bestehend aus
1) -H,
2) -(C₁-C₆)-Alkyl, worin Alkyl geradkettig oder verzweigtkettig ist,
3) -(C₁-C₆)-Alkenyl, worin Alkenyl geradkettig oder verzweigtkettig ist und gegebenenfalls bis zu drei Doppelbindungen enthält,
4) -Cycloalkyl, insbesondere C₃-C₈ Cycloalkyl,
5) -Aryl, insbesondere C₆-C₁₂ Aryl,
6) -(C₁-C₄)-Carboxyalkyl, falls es sich bei Verbindung I um Pyruvat handelt, gegebenenfalls auch Carboxyl.

Im offenbarten Verfahren können R₂ und R₃ auch unabhängig voneinander ausgewählt sein aus der Gruppe bestehend aus H, (C₁-C₆)-Alkyl, worin Alkyl geradkettig oder verzweigt ist, (C₁-C₆)-Alkenyl, worin Alkenyl geradkettig oder verzweigt ist und ein bis drei Doppelbindungen enthält, Aryl, insbesondere C₆-C₁₂ Aryl, Carboxyl, oder (C₁-C₄)-Carboxyalkyl.

Gegenüber dem Stand der Technik stellt ein Verfahren gemäß vorliegender Erfindung eine wesentliche Verbesserung von Verfahren dar, bei denen Verbindungen sowohl enzymatisch oxidiert als auch reduziert werden, da es damit ermöglicht wird, die nötigen Oxidations- und Reduktionsreaktionen sowie die zugehörigen Reaktionen zur Kofaktor-Regenerierung in einem Reaktionsansatz ablaufen zu lassen und gleichzeitig wesentlich höhere Substratkonzentrationen einzusetzen als dies im Stand der Technik der Fall ist.

In einem Verfahren gemäß vorliegender Erfindung werden die Kofaktoren NADH und NADPH eingesetzt. Dabei bezeichnet NAD⁺ die oxidierte Form und NADH die reduzierte Form von Nicotinamidadenindinucleotid während NADP⁺ die oxidierte Form und NADPH die reduzierte Form von Nicotinamidadenindinucleotidphosphat bezeichnen.

Als "Oxidationsreaktion(en)" und "Reduktionsreaktion(en)" werden hier diejenigen enzymkatalysierten Redoxreaktionen bezeichnet, die nicht Teil der Kofaktor-Regenerierung sind und in einem Verfahren gemäß vorliegender Erfindung an der Bildung des Produkts beteiligt sind. "Oxidationsreaktion(en)" und "Reduktionsreaktion(en)" sind unter dem Begriff "Produktbildungsreaktionen" zusammengefasst. Die Produktbildungsreaktionen in einem Verfahren gemäß vorliegender Erfindung beinhalten jeweils mindestens eine Oxidationsreaktion sowie mindestens eine Reduktionsreaktion.

Im erfindungsgemäßen Verfahren wird NAD⁺ als Kofaktor für die Oxidationsreaktion(en) verwendet und NADPH der Kofaktor für die Reduktionsreaktion(en). Offenbart wird hier auch ein Verfahren, in dem NADP⁺ als Kofaktor für die Oxidationsreaktion(en) und NADH als Kofaktor für die Reduktionsreaktionen verwendet wird. In einem Verfahren gemäß vorliegender Erfindung können Oxidationsreaktion(en) und Reduktionsreaktion(en) entweder zeitlich parallel oder zeitlich nacheinander, bevorzugt zeitlich parallel im selben Reaktionsansatz durchgeführt werden.

Als Substrate werden hier diejenigen Verbindungen bezeichnet, die mit dem Ziel der Produktbildung eingesetzt werden. Als Kosubstrate werden hier diejenigen Verbindungen bezeichnet, die bei der Kofaktor-Regenerierung umgesetzt werden.

In einem Verfahren gemäß vorliegender Erfindung können sowohl ein Substrat, als auch mehrere Substrate eingesetzt werden. Dabei können Reduktions- und/oder Oxidationsreaktion(en) sowohl am selben Substrat (Molekülgrundgerüst), als auch an verschiedenen Substraten, bevorzugt am selben Substrat, erfolgen. Weiterhin können in einem Verfahren gemäß vorliegender Erfindung Reduktions- und/oder Oxidationsreaktion an derselben oder an verschiedenen funktionellen Gruppen stattfinden.

Ein Verfahren gemäß vorliegender Erfindung eignet sich für eine Vielzahl von Reaktionen, beispielsweise zur Konfigurationsumkehr stereoisomerer Hydroxyverbindungen mittels Oxidation zum entsprechenden Keton und darauffolgender Reduktion zur entgegengesetzt stereospezifischen Hydroxyverbindung.

Unter "Ein-Topf-Reaktion" wird hier ein Verfahren bezeichnet, bei dem zwei oder mehr an der Produktbildung beteiligte enzymatische Redoxreaktionen und zwei enzymatische Systeme zur Kofaktor-Regenerierung in einem Reaktionsansatz ablaufen, ohne dass ein Zwischenprodukt isoliert wird.

Die Nennung einer Säure oder des Salzes einer Säure schließt hier den jeweils nicht genannten Begriff ein. Ebenfalls schließt die Nennung von Säuren, insbesondere Gallensäuren, hier alle davon abgeleiteten Ester mit ein. Weiter sind hier (partiell) mit Schutzgruppen versehene Verbindungen bei der Nennung der zugrundeliegenden Substanzen mit eingeschlossen.

In einer bevorzugten Ausführungsform der vorliegenden Erfindung ist ein Verfahren gemäß vorliegender Erfindung dadurch gekennzeichnet, dass Oxidationsreaktion und Reduktionsreaktion zeitlich parallel ablaufen.

In einer bevorzugten Ausführungsform der vorliegenden Erfindung ist ein Verfahren gemäß vorliegender Erfindung dadurch gekennzeichnet, dass sowohl Oxidationsreaktion als auch Reduktionsreaktion am selben Molekülgrundgerüst stattfinden.

In einer bevorzugten Ausführungsform der vorliegenden Erfindung ist ein Verfahren gemäß vorliegender Erfindung dadurch gekennzeichnet, dass in a) Pyruvat (Kosubstrat) eingesetzt wird, welches mittels einer Laktatdehydrogenase zu Laktat reduziert wird, das heisst, dass bei der Regenerierungsreaktion, die den reduzierten Kofaktor wieder in seine ursprüngliche oxidierte Form überführt mittels einer Laktatdehydrogenase Pyruvat zu Laktat reduziert wird.

In einer bevorzugten Ausführungsform der vorliegenden Erfindung ist ein Verfahren gemäß vorliegender Erfindung dadurch gekennzeichnet, dass in b) 2-Propanol (Isopropylalkohol, IPA) (Kosubstrat) eingesetzt wird, welches mittels einer Alkoholdehydrogenase zu Aceton oxidiert wird, das heisst, dass bei der Regenerierungsreaktion, die den oxidierten Kofaktor wieder in seine ursprüngliche reduzierte Form überführt mittels einer Alkoholdehydrogenase 2-Propanol zu Aceton oxidiert wird.

In einer bevorzugten Ausführungsform der vorliegenden Erfindung ist ein Verfahren gemäß vorliegender Erfindung dadurch gekennzeichnet, dass Sauerstoff eingesetzt wird, welcher mittels einer NADH Oxidase reduziert wird.

In einer bevorzugten Ausführungsform der vorliegenden Erfindung ist ein Verfahren gemäß vorliegender Erfindung dadurch gekennzeichnet, dass in b) Malat (Kosubstrat) eingesetzt wird, welches mittels einer Oxalacetat-decarboylierenden Malatdehydrogenase ("Malatenzym") zu Pyruvat und CO₂ oxidiert wird, z.B. dass bei der Regenerierungsreaktion, die den oxidierten Kofaktor wieder in seine ursprüngliche reduzierte Form überführt mittels einer Malatdehydrogenase Malat zu Pyruvat und CO₂ oxidiert wird.

Das entstehende Pyruvat wird bei dieser Ausführungsform in einer weiteren Redoxreaktion umgesetzt, die nicht zur Produktbildung dient, sondern die zweite Kofaktor-Regenerierungsreaktion darstellt.

In einer bevorzugten Ausführungsform der vorliegenden Erfindung ist ein Verfahren gemäß vorliegender Erfindung dadurch gekennzeichnet, dass es eingesetzt wird zur Durchführung von jeweils mindestens einer Oxidationsreaktion und mindestens einer Reduktionsreaktion im selben Reaktionsansatz an Verbindungen der allgemeinen Formel 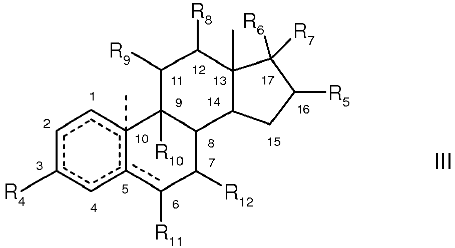 worin
R₄ Wasserstoff, eine Methylgruppe, eine Hydroxygruppe oder eine Oxogruppe,
R₅ Wasserstoff, eine Hydroxygruppe, eine Oxogruppe oder eine Methylgruppe,
R₆ Wasserstoff oder eine Hydroxygruppe,
R₇ Wasserstoff, -COR₁₃, worin R₁₃ eine unsubstituierte oder mit einer Hydroxygruppe substituierte C₁-C₄-Alkylgruppe ist, oder eine substituierte, insbesondere mit einer Hydroxygruppe, oder unsubstituierte C₁-C₄ Carboxyalkylgruppe,
oder R₆ und R₇ zusammen eine Oxogruppe bedeuten,
R₈ Wasserstoff, eine Methylgruppe, eine Hydroxygruppe oder eine Oxogruppe,
R₉ Wasserstoff, eine Methylgruppe, eine Hydroxygruppe oder eine Oxogruppe,
R₁₀ Wasserstoff, eine Methylgruppe oder ein Halogen,
R₁₁ Wasserstoff, eine Methylgruppe, eine Hydroxygruppe, eine Oxogruppe oder Halogen, und
R₁₂ Wasserstoff, eine Hydroxygruppe, eine Oxogruppe oder eine Methylgruppe bedeuten, wobei das Strukturelement 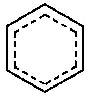
einen Benzolring oder einen Ring mit 6 Kohlenstoffatomen und 0, 1 oder 2 C-C-Doppelbindungen bedeutet;
wobei bevorzugt das Substrat/die Substrate für die an der Produktbildung beteiligte(n) Reduktionsreaktion(en) in einer Konzentration von <5% (w/v) im Reaktionsansatz vorliegt/vorliegen.

In einer bevorzugten Ausführungsform der vorliegenden Erfindung ist ein Verfahren gemäß vorliegender Erfindung dadurch gekennzeichnet, dass eine enzymatische Umwandlung von Dehydroepiandrosteron (DHEA) 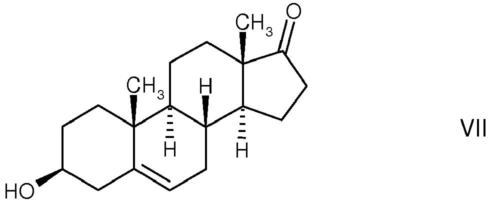 in Testosteron der Formel 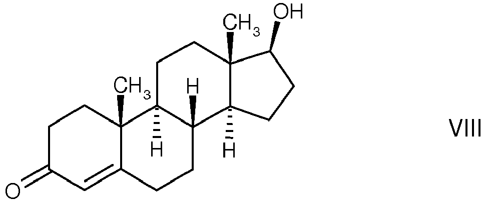 erfolgt.

In einer bevorzugten Ausführungsform der vorliegenden Erfindung ist ein Verfahren gemäß vorliegender Erfindung dadurch gekennzeichnet, dass es zur enzymatischen Epimerisierung der Hydroxysteroidverbindung 3α,7α-Dihydroxy-5β-cholansäure (Chenodeoxycholsäure, CDC) der Formel 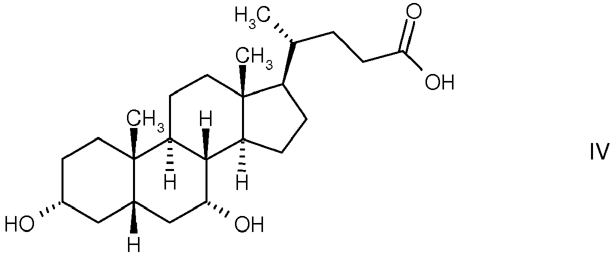 durch Oxidation zu Ketolithocholsäure (KLC) der Formel 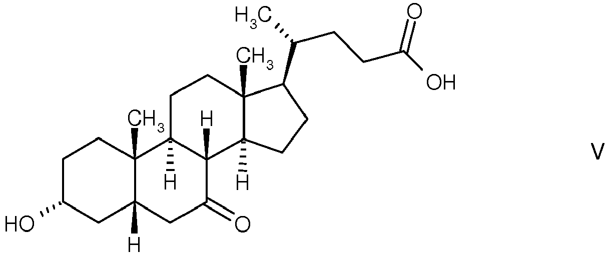 und nachfolgende Reduktion zur stereoisomeren Hydroxyverbindung 3α,7β-Dihydroxy-5β-cholansäure (Ursodeoxycholsäure) der Formel 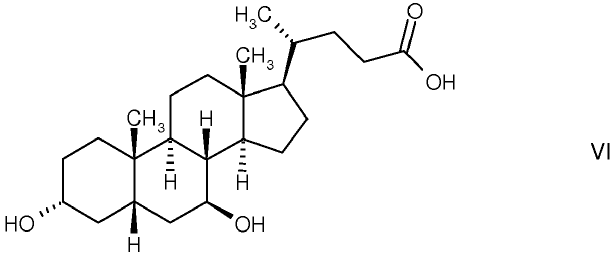 mittels zweier entgegengesetzt stereospezifischer Hydroxysteroiddehydrogenasen erfolgt.

In einer bevorzugten Ausführungsform der vorliegenden Erfindung ist ein Verfahren gemäß vorliegender Erfindung dadurch gekennzeichnet, dass eine enzymatischen Epimerisierung von 3α,7α,12α-Trihydroxy-5β-cholansäure (Cholsäure) der Formel 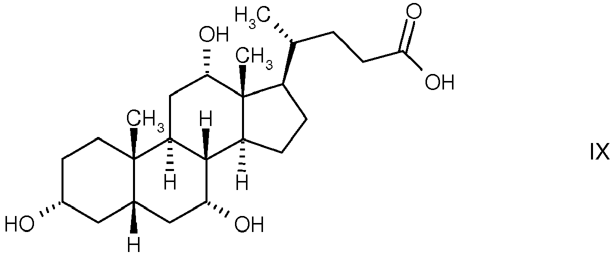 **entweder**
A) durch Oxidation zu 3α,7α-Dihydroxy-12-oxo-5β-cholansäure (12-oxo-CDC) der Formel 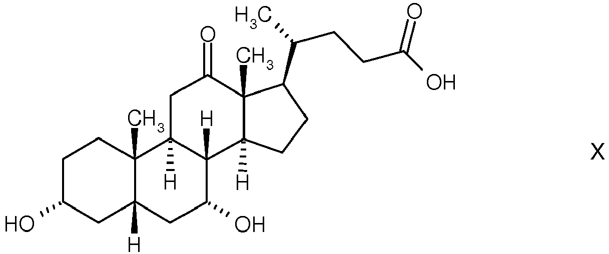 die weiter umgesetzt wird zur 3α-Hydroxy-7,12-dioxo-5β-cholansäure (12oxo-KLC) der Formel 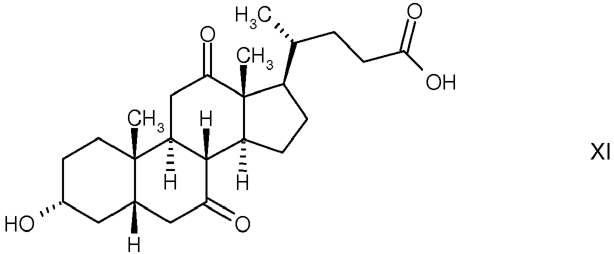 und nachfolgende Reduktion zur stereoisomeren Hydroxyverbindung 3α,7β-Dihydroxy-12-oxo-5β-cholansäure (12-Keto-Ursodeoxycholsäure) der Formel 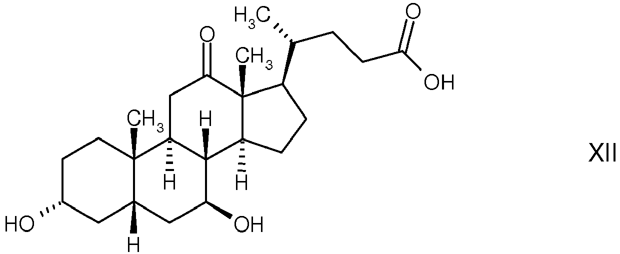 oder
B) durch Oxidation zu 3α,12α-Dihydroxy-7-oxo-5β-cholansäure der Formel Formel 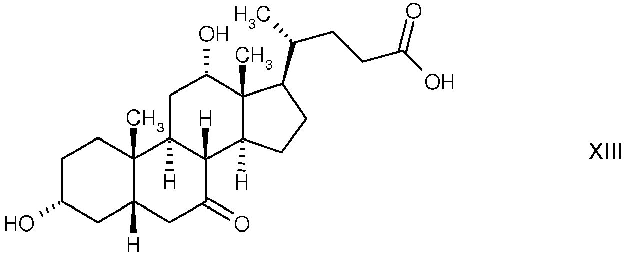 gefolgt von enzymatischer Oxidation zur 3α-Hydroxy-7,12-dioxo-5β-cholansäure (12oxo-KLC) der Formel XI, und nachfolgende Reduktion zur stereoisomeren Hydroxyverbindung 3α,7β-Dihydroxy-12-oxo-5β-cholansäure (12-Keto-Ursodeoxycholsäure) der Formel XII,
   **oder**
C) durch Oxidation zu 3α,12α-Dihydroxy-7-oxo-5β-cholansäure der Formel XIII, gefolgt von enzymatischer Reduktion zur 3α,7β,12α-Triydroxy-5β-cholansäure der Formel 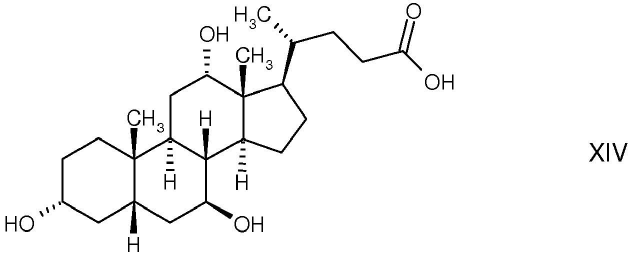 und nachfolgende Oxidation zur stereoisomeren Hydroxyverbindung 3α,7β-Dihydroxy-12-oxo-5β-cholansäure (12-Keto-Ursodeoxycholsäure) der Formel XII;
**oder**
in irgendeiner Kombination aus A), B) und/oder C;
z.B. mittels dreier stereospezifischer Hydroxysteroiddehydrogenasen; wobei 2 der drei stereospezifischer Hydroxysteroiddehydrogenasen entgegengesetzt spezifisch sind;

In einer bevorzugten Ausführungsform der vorliegenden Erfindung ist ein Verfahren gemäß vorliegender Erfindung dadurch gekennzeichnet, dass ein C₅- oder C₆-Zucker als Substrat eingesetzt wird, z.B. dass das Verfahren zur Isomerisierung von C₅- oder C₆-Zuckern eingesetzt wird.

In einer bevorzugten Ausführungsform der vorliegenden Erfindung ist ein Verfahren gemäß vorliegender Erfindung dadurch gekennzeichnet, dass eine Isomerisierung von Glukose durch Reduktion zu Sorbitol und Oxidation zu Fruktose erfolgt, z.B. dass das Verfahren eingesetzt wird zur Isomerisierung von Glukose durch Reduktion zu Sorbitol und nachfolgende Oxidation zu Fruktose.

Ein Verfahren gemäß vorliegender Erfindung wird bevorzugt in einem wässrigen System durchgeführt, wobei es möglich ist, dass das Substrat für Oxidations- und Reduktionsreaktion zum Teil ungelöst in Form einer Suspension und/oder als zweite flüssige Phase vorliegt.

In einer besonderen Ausführungsform ist ein Verfahren gemäß vorliegender Erfindung dadurch gekennzeichnet, dass das Substrat/die Substrate für die an der Produktbildung beteiligte(n) Oxidationsreaktion(en) in einer Konzentration von mindestens 5% (w/v) und mehr, bevorzugt 7% (w/v) und mehr, insbesondere bevorzugt 9% (w/v) und mehr im Reaktionsansatz vorliegt/vorliegen, z.B. 5% (w/v) bis 20% (w/v), wie 5% (w/v) bis 15% (w/v), z.B. 5% (w/v) bis 12% (w/v), wie 5% (w/v) bis 10% (w/v).

In einer besonderen Ausführungsform ist ein Verfahren gemäß vorliegender Erfindung dadurch gekennzeichnet, dass bei den Produktbildungsreaktionen insgesamt ein Umsatz von ≥70%, insbesondere ≥90% erreicht wird.

In einem Verfahren gemäß vorliegender Erfindung kann dem wässrigen System ein Puffer zugesetzt werden. Geeignete Puffer sind zum Beispiel Kaliumphosphat, Tris-HCl und Glycin mit einem pH-Wert von 5 bis 10, vorzugsweise von 6 bis 9. Weiters, oder alternativ können dem System Ionen zur Stabilisierung der Enzyme, wie zum Beispiel Mg²⁺ oder sonstige Zusätze, wie zum Beispiel Glycerin zugesetzt werden. Die Konzentration der zugesetzten Kofaktoren NAD(P)⁺ und NAD(P)H beträgt in einem Verfahren gemäß vorliegender Erfindung üblicherweise zwischen 0,001 mM und 10 mM, vorzugsweise zwischen 0,01 mM und 1 mM.

Abhängig von den verwendeten Enzymen kann das Verfahren gemäß vorliegender Erfindung bei einer Temperatur von 10°C bis 70°C, vorzugsweise von 20°C bis 45°C durchgeführt werden.

Unter Hydroxysteroiddehydrogenasen (HSDH) versteht man solche Enzyme, die die Oxidation von Hydroxygruppen zu den entsprechenden Ketogruppen oder umgekehrt die Reduktion von Ketogruppen zu den entsprechenden Hydroxygruppen am Steroidgerüst katalysieren.

Geeignete Hydroxysteroiddehydrogenasen, die für Redoxreaktionen an Hydroxysteroiden eingesetzt werden können, sind zum Beispiel 3α-HSDH, 3β-HSDH, 7α-HSDH, 7β-HSDH oder 17β-HSDH.

Geeignete Enzyme mit 7α-HSDH-Aktivität sind zum Beispiel erhältlich aus Clostridien (*Clostridium absonum, Clostridium sordelii*), *Escherichia coli* oder *Bacteroides fragilis.*

Geeignete Enzyme mit 7β-HSDH-Aktivität sind zum Beispiel erhältlich aus *Ruminococcus sp.* oder *Clostridium absonum.*

Geeignete Laktatdehydrogenasen sind zum Beispiel erhältlich aus *Oryctolagus cuniculus.*

Geeignete Alkoholdehydrogenasen sind zum Beispiel erhältlich aus *Lactobacillus kefir.*

Eine geeignete Xylosereduktase ist zum Beispiel erhältlich aus *Candida tropicalis.*

Geeignete Sorbitoldehydrogenasen sind zum Beispiel erhältlich aus Schafsleber, *Bacillus subtilis* oder *Malus domestica.*

Geeignete NADH Oxidasen sind zum Beispiel erhältlich aus *Leuconostoc mesenteroides, Streptococcus mutans, Clostridium aminovalericum.*

Enzyme werden in einem Verfahren gemäß vorliegender Erfindung bevorzugt als in *E. coli* rekombinant überexprimierte Proteine verwendet, wobei weiterhin bevorzugt die entsprechenden Zell-Lysate ohne weitere Aufreinigung eingesetzt werden. Die Enzym-Einheit 1 U entspricht dabei derjenigen Enzymmenge, die benötigt wird, um 1 µmol Substrat pro min umzusetzen.

### Beschreibung der Abbildungen

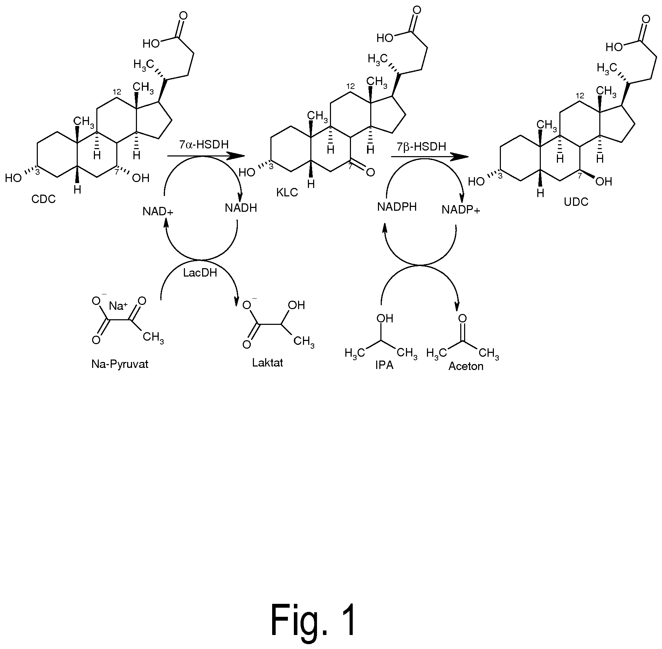

Fig. 1 zeigt das Reaktionsschema der Epimerisierung von Chenodeoxycholsäure zu Ursodeoxycholsäure über das Zwischenprodukt 3α-Hydroxy-7oxo-5β-cholansäure mit Kofaktor-Regenerierung unter Verwendung von 2-Propanol und Pyruvat.

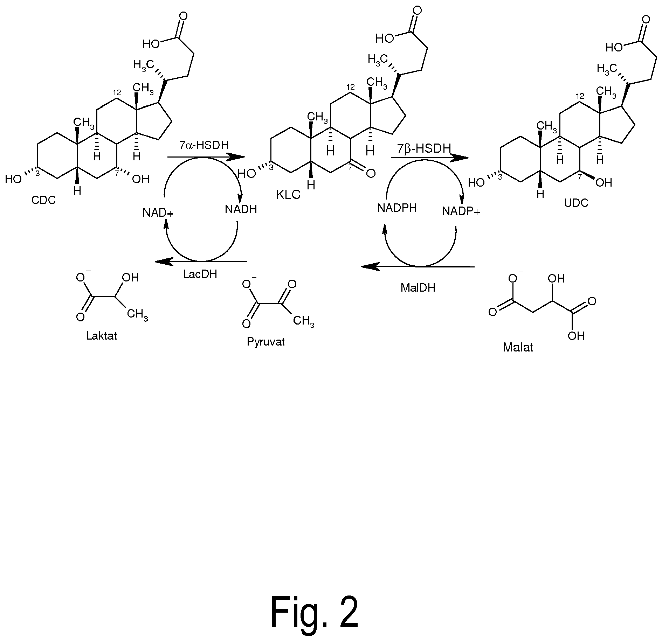

Fig. 2 zeigt das Reaktionsschema der Epimerisierung von Chenodeoxycholsäure zu Ursodeoxycholsäure über das Zwischenprodukt 3α-Hydroxy-7oxo-5β-cholansäure mit Kofaktor-Regenerierung unter Verwendung von Malat und Pyruvat.

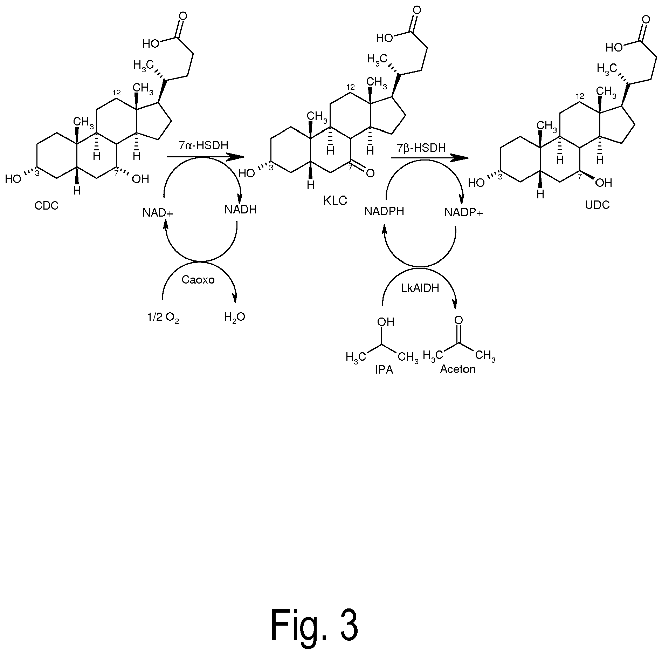

Fig. 3 zeigt das Reaktionsschema der Epimerisierung von Chenodeoxycholsäure zu Ursodeoxycholsäure über das Zwischenprodukt 3α-Hydroxy-7oxo-5β-cholansäure mit Kofaktor-Regenerierung unter Verwendung von 2-Propanol und Sauerstoff.

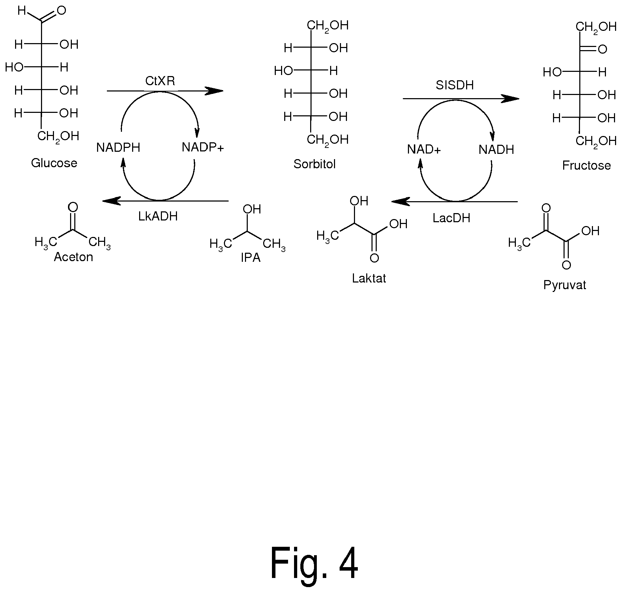

Fig. 4 zeigt das Reaktionsschema der Isomerisierung von Glukose zu Fruktose mit Kofaktor-Regenerierung unter Verwendung von 2-Propanol und Pyruvat.

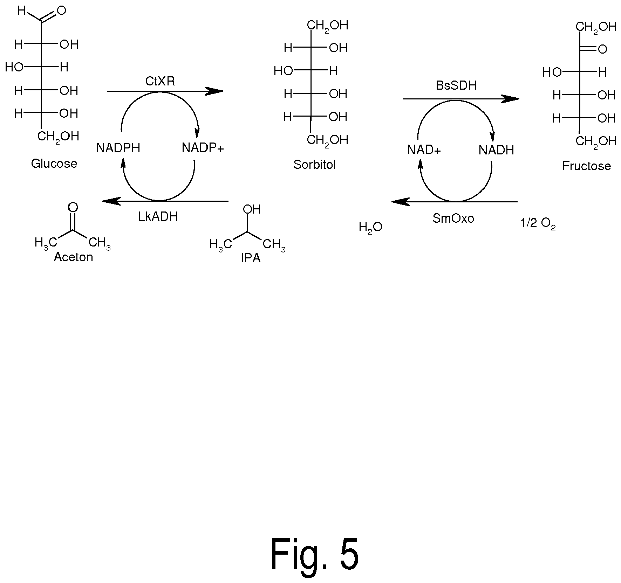

Fig. 5 zeigt das Reaktionsschema der Isomerisierung von Glukose zu Fruktose mit Kofaktor-Regenerierung unter Verwendung von 2-Propanol und Sauerstoff.

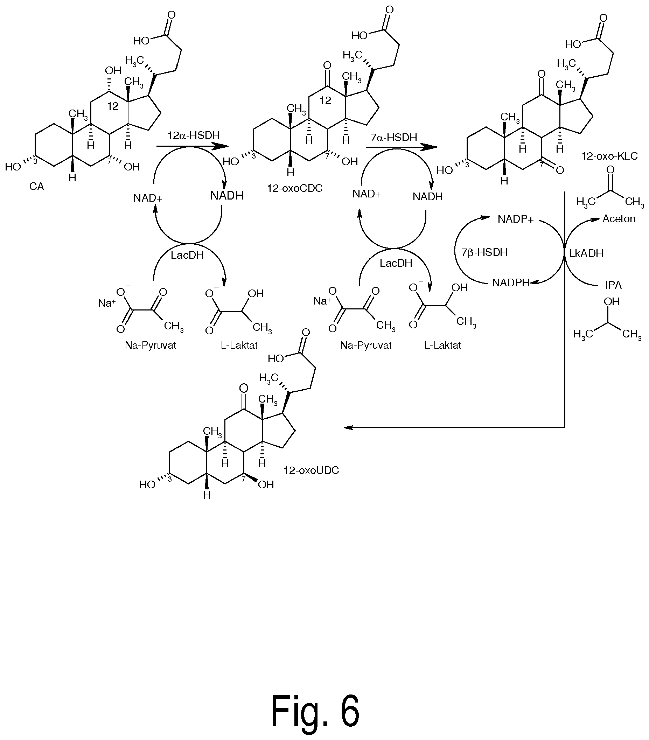

Fig. 6 zeigt das Reaktionsschema der Epimerisierung von Cholsäure zu 3α,7β-Dihydroxy-12-oxo-5β-cholansäure über die Zwischenprodukte 3α,7α-Dihydroxy-12-oxo-5β-cholansäure und 3α-Hydroxy-7,12-dioxo-5β-cholansäure mit Kofaktor-Regenerierung unter Verwendung von 2-Propanol und Pyruvat.

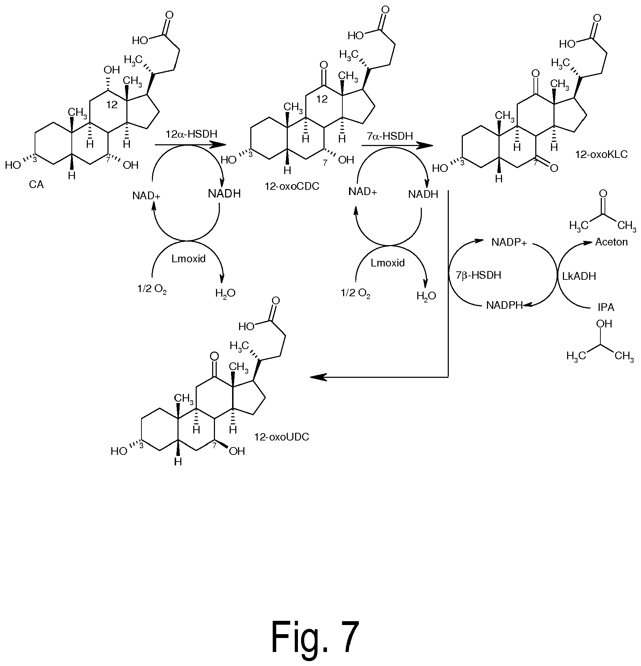

Fig. 7 zeigt das Reaktionsschema der Epimerisierung von Cholsäure zu 3α,7β-Dihydroxy-12-oxo-5β-cholansäure über die Zwischenprodukte 3α,7α-Dihydroxy-12-oxo-5β-cholansäure und 3α-Hydroxy-7,12-dioxo-5β-cholansäure mit Kofaktor-Regenerierung unter Verwendung von 2-Propanol und Sauerstoff.

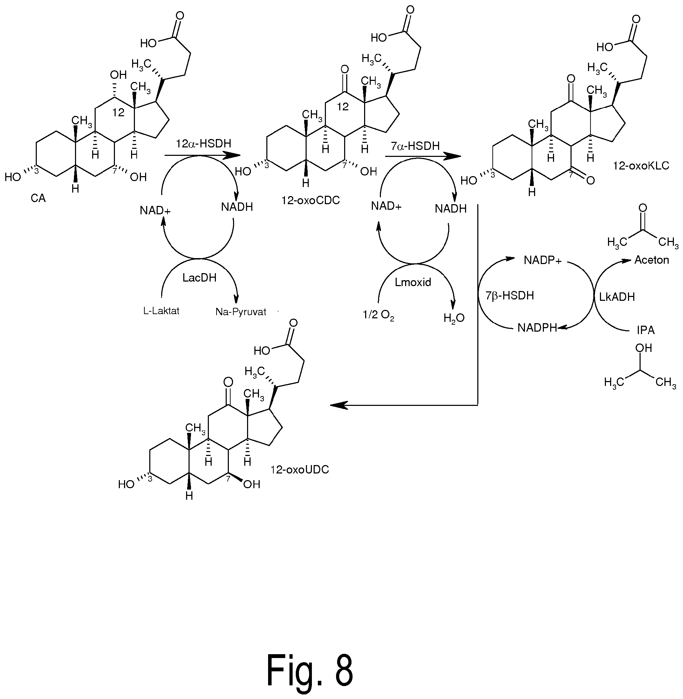

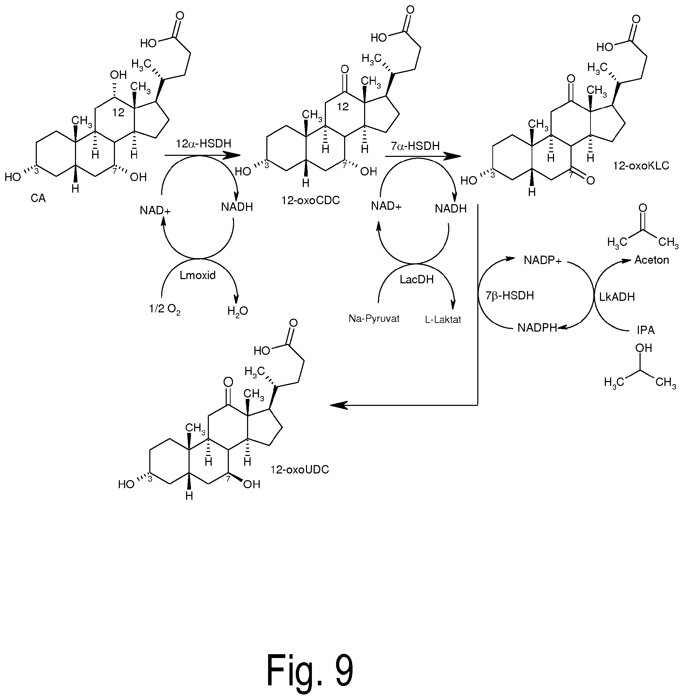

Fig. 8 und Fig. 9 zeigen Reaktionsschemata der Epimerisierung von Cholsäure zu 3α,7β-Dihydroxy-12-oxo-5β-cholansäure über die Zwischenprodukten 3α,7α-Dihydroxy-12-oxo-5β-cholansäure und 3α-Hydroxy-7,12-dioxo-5β-cholansäure mit Kofaktor-Regenerierung unter Verwendung von 2-Propanol, Pyruvat und Sauerstoff.

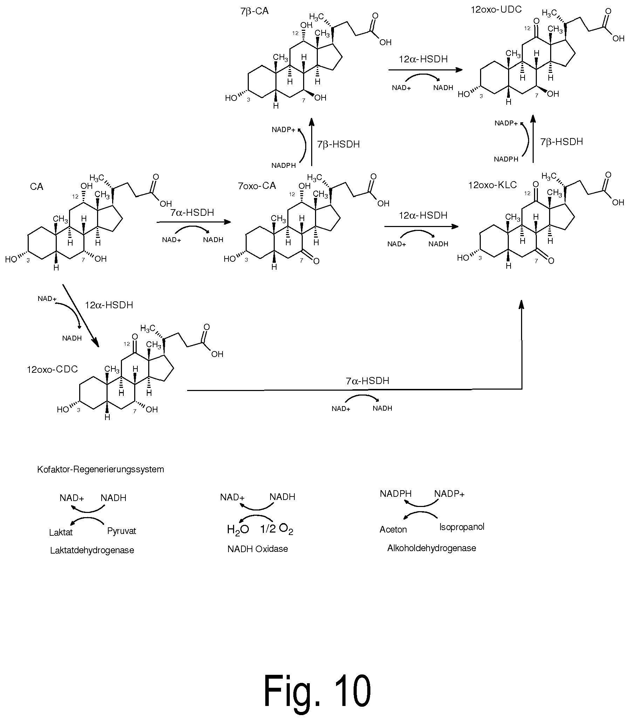

Fig. 10 zeigt die möglichen Reaktionswege der Epimerisierung von Cholsäure zu 3α,7β-Dihydroxy-12-oxo-5β-cholansäure über verschiedene Zwischenprodukten mit Kofaktor-Regenerierungssystemen. Um NAD⁺ wiederherzustellen wurden abwechselnd Laktatdehydrogenase (Pyruvat als Substrat) und NADH Oxidase (Sauerstoff als Substrat) eingesetzt. Um NADPH zu regenerieren wurde Alkoholdehydrogenase (Isopropanol als Substrat) eingesetzt.

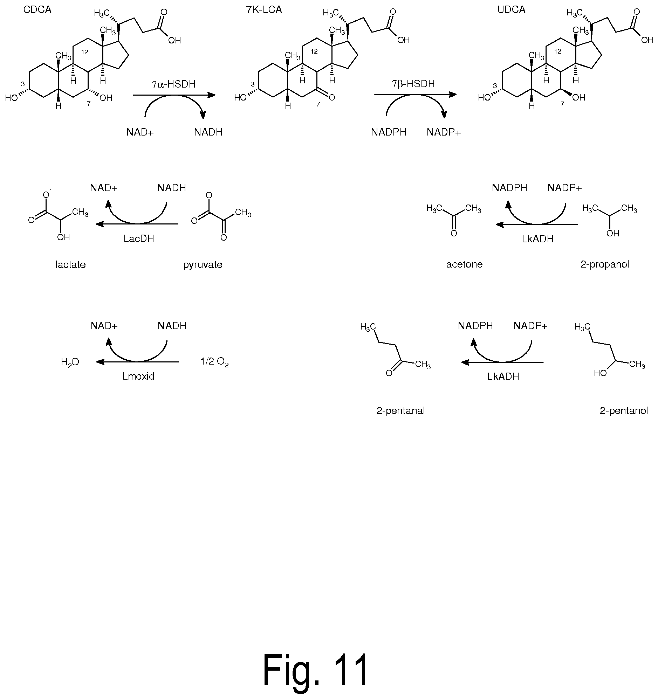

Fig. 11 zeigt das Reaktionsschema der Epimerisierung von Chenodeoxycholsäure zu Ursodeoxycholsäure über das Zwischenprodukt 3α-Hydroxy-7oxo-5β-cholansäure (7-Ketolithocholsäure = 7K-LCA = KLC) mit Kofaktor-Regenerierung unter Verwendung von 2-Propanol und 2-Pentanol (jeweils Alkoholdehydrogenase) sowie Pyruvat (Laktat-Dehydrogenase) und Sauerstoff (NADH-Oxidase).

In den Abbildungen werden die folgenden Abkürzungen verwendet:
- BsSDH: = Sorbitol-Dehydrogenase aus Bacillus subtilis
- CA: = 3α,7α,12α-Trihydroxy-5β-cholansäure
- 7β-CA: = 3α,7β,12α,-Trihydroxy-5β-cholansäure
- Caoxo: = *Clostridium aminovalericum* NADH Oxidase
- CDC: = 3α,7α-Dihydroxy-5β-cholansäure
- CDCA: = 3α,7α-Dihydroxy-5β-cholansäure
- CtXR: = *Candida tropicalis* Xylosereduktase
- 7α-HSDH: = 7α-Hydroxysteroiddehydrogenase
- 7β-HSDH: 7β-Hydroxysteroid dehydrogenase
- 12α-HSDH: = 12α-Hydroxysteroiddehydrogenase
- KLC: = 3α-Hydroxy-7-oxo- 5β-cholansäure
- 7K-LCA: = 3α-Hydroxy-7-oxo- 5β-cholansäure
- LacDH: = Laktatdehydrogenase NAD(H)-abhängig
- LkADH: = *Lactobacillus kefir* Alkoholdehydrogenase NADP(H)-abhängig
- Lmoxid: = *Leuconostoc mesenteroides* NADH-Oxidase
- MalDH: = *E. coli* Malatdehydrogenase NADP(H)-abhängig
- 7oxo-CA: = 3α,12 α-Dihydroxy-7-oxo-5β-cholansäure
- 12oxo-CDC: = 3α,7α-Dihydroxy-12-oxo-5β-cholansäure
- 12oxo-KLC: = 3α-Hydroxy-7,12-dioxo-5β-cholansäure
- 12oxo-UDC: = 3α,7β-Dihydroxy-12-oxo-5β-cholansäure
- SISDH: = Schafsleber-Sorbitoldehydrogenase
- SmOxo: = *Streptococcus mutans* NADH Oxidase
- UDC: = 3α,7β-Dihydroxy-5β-cholansäure
- UDCA: = 3α,7β-Dihydroxy-5β-cholansäure

In den nachfolgenden Beispielen sind alle Tempreaturangaben in Grad Celsius (°C). Die folgenden Abkürzungen werden verwendet:
- EtOAc: Ethylacetat
- h: Stunde(n)
- IPA: Isopropylalkohol (2-Propanol)
- MeOH: Methanol
- Rt: Raumtemperatur

### Beispiel 1

### Epimerisierung von Chenodeoxycholsäure zu Ursodeoxycholsäure durch 7α-Hydroxysteroiddehydrogenase und 7β-Hydroxysteroiddehydrogenase unter der Verwendung eines Laktatdehydrogenase- und Alkoholdehydrogenase-abhängigen Kofaktor-Regenerierungssystems

Ein 0,5 ml-Ansatz enthält 50 mg Chenodeoxycholsäure, 12 U der rekombinanten 7α-Hydroxysteroiddehydrogenase aus *Escherichia coli,* 6 U der rekombinanten 7β-Hydroxysteroiddehydrogenase aus *Ruminococcus torques* sowie 0,5 mM NAD⁺ und 0,3 mM NADPH. Zur Regenerierung von NAD⁺ werden 6 U rekombinante Laktatdehydrogenase und 350 mM Natrium-Pyruvat eingesetzt. Für die Regenerierung von NADPH werden 6 U der rekombinanten Alkoholdehydrogenase aus *Lactobacillus kefir* und anfänglich 2.4% IPA (w/v) eingesetzt. Die Reaktion wird in einem wässrigen Kaliumphosphatpuffer (100 mM, pH = 7,8) bei 25°C und unter kontinuierlichem Schütteln (850 rpm) durchgeführt. Es wird weiterhin ein offenes System verwendet, um die Verdampfung von Aceton zu ermöglichen und die Reaktion in Richtung Ursodeoxycholsäure zu verschieben. Es werden 1,6% (w/v) IPA nach 6 h, 2,4% (w/v) IPA nach 16 h, 3,9% (w/v) IPA nach 24 h und 0,8% (w/v) IPA nach 40 h nachdosiert. Außerdem werden nach 24 h 20 µl 4-Methyl-2-pentanol zugegeben. Nach 46 h werden 200 µl 2-Pentanol, sowie 1,6% (w/v) IPA zugegeben. Nach 48 h beträgt der Anteil von Ursodeoxycholsäure an allen Gallensäuren in der Reaktionsmischung >97%.

### Beispiel 2

### Epimerisierung von Chenodeoxycholsäure zu Ursodeoxycholsäure durch 7α-Hydroxysteroiddehydrogenase und 7β-Hydroxysteroiddehydrogenase unter der Verwendung eines Laktatdehydrogenase- und Malatdehydrogenase-abhängigen Kofaktor-Regenerierungssystems

Ein 0,5 ml-Ansatz enthält 50 mg Chenodeoxycholsäure, 20 U der rekombinanten 7α-Hydroxysteroiddehydrogenase aus *Escherichia coli,* 20 U der rekombinanten 7β-Hydroxysteroiddehydrogenase aus *Ruminococcus torques* sowie 1 mM NAD⁺ und 1 mM NADPH.

Zur Regenerierung von NAD⁺ werden 10 U der Laktatdehydrogenase (Sigma-Aldrich) und zum Start der Reaktion 16,5 mM Natrium-Pyruvat eingesetzt. Für die Regenerierung von NADPH werden 20 U der rekombinanten Malatdehydrogenase aus *Escherichia coli* und 320 mM Natrium-Malat eingesetzt. Die Reaktion wird in einem wässrigen Kaliumphosphatpuffer (100 mM, pH = 7,8) bei 25°C und unter kontinuierlichem Schütteln (850 rpm) durchgeführt. Es wird weiterhin ein offenes System verwendet, um ein Entweichen des entstehenden CO₂ zu ermöglichen. Es wurden 20 U 7α-HSDH sowie 10 U Laktatdehydrogenase nach 16 h und 40 h nachdosiert. Es wurden 10 U 7β-HSDH nach 20 h, 24 h, 44 h und 48 h nachdosiert. Weiterhin wurden 10 U Malatdehydrogenase nach 40 h nachdosiert. Nach 72 h beträgt der Anteil von Ursodeoxycholsäure an allen Gallensäuren in der Reaktionsmischung ca. 90%.

### Beispiel 3

### Epimerisierung von Chenodeoxycholsäure zu Ursodeoxycholsäure durch 7α-Hydroxysteroiddehydrogenase und 7β-Hydroxysteroiddehydrogenase unter der Verwendung eines NADH Oxidase- und Alkoholdehydrogenase-abhängigen Kofaktor-Regenerierungssystems

Ein 0,5 ml-Ansatz enthält 50 mg Chenodeoxycholsäure, 12 U der rekombinanten 7α-Hydroxysteroiddehydrogenase aus *Escherichia coli*, 7,5 U der rekombinanten 7β-Hydroxysteroiddehydrogenase aus *Ruminococcus torques* sowie 1 mM NAD⁺ und 1 mM NADPH. Zur Regenerierung von NAD⁺ werden 20 U der rekombinanten NADH-Oxidase aus *Clostridium aminovalericum* eingesetzt. Für die Regenerierung von NADPH werden 5 U der rekombinanten Alkoholdehydrogenase aus *Lactobacillus kefir* und anfänglich 2 % IPA (w/v) eingesetzt. Die Reaktion wird in einem wässrigen Kaliumphosphatpuffer (100 mM, pH 6) bei 25°C und unter kontinuierlichem Schütteln (850 rpm) durchgeführt. Es wird weiterhin ein offenes System verwendet, um die Verdampfung von Aceton zu ermöglichen und die Reaktion in Richtung Ursodeoxycholsäure zu verschieben. Es werden 2% IPA nach 18 h, 22 h, 26 h und 41 h sowie 5% IPA nach 41 h und 48 h nachdosiert. Nach 24 h werden 20 U NADH-Oxidase und nach 41 h 7,5 U 7β-Hydroxysteroiddehydrogenase sowie 5 U Alkoholdehydrogenase nachdosiert. Nach 48 h beträgt der Anteil von Ursodeoxycholsäure an allen Gallensäuren in der Reaktionsmischung ca. 95-98%.

### Beispiel 4

### Aufarbeitung und Analytik der Gallensäuren

Nach Beendigung von Reaktionen, wie sie in den Beispielen 1 bis 3 beschrieben sind, wird das Reaktionsgemisch mit EtOAc extrahiert. Das Lösungsmittel wird anschließend mittels Abdampfens entfernt. Der Abdampfrückstand wird in einer Mischung von MeOH:Acetonitril:Natriumphosphatpuffer pH = 3, 0,78 g/l (40:30:37) gelöst und die Umsetzung der Chenodeoxycholsäure zu Ursodeoxycholsäure wird mittels HPLC verfolgt. Dabei wird eine Reversed-Phase-Trennsäule (ZORBAX®Eclipse® XDB C18, Fluss 0,8 ml/min) und ein Lichtbrechungsdetektor (RID), Agilent 1260 Infinity®, beide von Agilent Technologies Inc., benutzt.

### Beispiel 5

### Umsatz von Glucose zu Fructose durch eine Xylosereduktase und eine Sorbitoldehydrogenase unter der Verwendung einer Alkoholdehydrogenase zum Recycling des NADPH und einer Laktatdehydrogenase zum Recycling des NAD⁺

Ein 0,5 ml Ansatz enthält 50 mg/ml Glucose und 6 U/ml der rekombinanten Xylosereduktase aus *Candida tropicalis* (überexprimiert in *E.coli* BL21 (DE3)) und 0,1 mM NADP⁺. Zur Regeneration des Kofaktors werden 7% (v/v) IPA und und 6 U/ml der rekombinanten Alkoholdehydrogenase aus *Lactobacillus kefir* (überexprimiert in *E.coli* BL21 (DE3)) zugefügt. Die Enzyme werden in Form von Zelllysat eingesetzt. Die Reaktion findet für 24 h bei 40° C und pH = 9 (50 mM Tris HCl-Puffer) unter kontinuierlichem Schütteln (900 rpm) in einem offenem System statt. Das offene System führt zur Entfernung des Acetons, was die Reaktion in Richtung Sorbitol-Bildung treibt. Im offenen System verdampfen Wasser und IPA ebenfalls, sodass diese nach 6 h und 21 h nachdosiert werden. Dabei wird jeweils wieder ein Gesamtvolumen von 0,5 ml, sowie eine IPA-Konzentration von 7% (v/v) eingestellt. Nach 24 h wird das Reaktionsgefäß bei 60°C unter Vakuum inkubiert, um die Enzyme zu deaktivieren und die organischen Lösungsmittel zu verdampfen. Nach dem Abkühlen auf Rt werden die rekombinante Sorbitoldehydrogenase aus *Bacillus subtilis* (überexprimiert in *E.coli* BL21 (DE3) in einer Endkonzentration von 5 U/ml, ZnCl₂ in einer Endkonzentration von 1 mM und NAD⁺ in einer Endkonzentration von 0,1 mM zugefügt. Zur Kofaktor-Regenerierung werden 5 U/ml (Endkonzentration) Laktat-Dehydrogenase aus Kaninchenmuskel (Sigma Aldrich) und 300 mM Pyruvat eingesetzt. Der Ansatz wird auf 0,5 ml mit Wasser aufgefüllt. Die Reaktion findet für weitere 24 h bei 40°C unter kontinuierlichem Schütteln (900 rpm) im geschlossenem System statt. Es wird ein Umsatz der D-Glucose zu D-Fructose von >90% erreicht.

### Beispiel 6

### Umsatz der Glucose zu Fructose durch eine Xylosereduktase und eine Sorbitoldehydrogenase unter der Verwendung einer Alkoholdehydrogenase zum Recycling des NADPH und einer Oxidase zum Recycling des NAD⁺

Ein 0,5 ml Ansatz enthält 50 mg/ml Glucose, 6 U/ml der rekombinanten Xylosereduktase aus *Candida tropicalis* (überexprimiert in *E.coli* BL21 (DE3) und 0,1 mM NADP⁺. Zur Regeneration des Kofaktors werden 7% (v/v) IPA und die rekombinante Alkoholdehydrogenase aus *Lactobacillus kefir* (überexprimiert in *E.coli* BL21 (DE3) zugefügt. Die Enzyme werden in Form von Zelllysat eingesetzt. Die Reaktion findet für 24 h bei 40°C und pH = 8 (50 mM Tris-HCl-Puffer) unter kontinuierlichem Schütteln (900 rpm) in einem offenem System statt. Das offene System führt zur Entfernung des Acetons, was die Reaktion Richtung Sorbitol-Bildung treibt. Im offenen System verdampfen Wasser und IPA ebenfalls, sodass diese nach 6 h und 21 h nachdosiert werden. Dabei wird jeweils wieder ein Gessamtvolumen von 0,5 ml und eine IPA-Konzentration von 7% (v/v) eingestellt.

Nach 24 h wird das Reaktionsgefäß bei 60°C unter Vakuum inkubiert, um die Enzyme zu deaktivieren und IPA, sowie entstandenes Aceton zu verdampfen. Nach dem Abkühlen auf Raumtemperatur werden die rekombinante Sorbitoldehydrogenase aus *Bacillus subtilis* (überexprimiert in *E.coli* BL21 (DE3) in einer Endkonzentration von 5 U/ml, CaCl₂ in einer Endkonzentration von 1 mM und eine Mischung aus NAD⁺ und NADH in einer Endkonzentration von 0,1 mM zugefügt. Zur Kofaktor-Regenerierung werden 10 U/ml (Endkonzentration) der NADH-Oxidase aus *Leuconostoc mesenteroides* überexprimiert in *E.coli* BL21 (DE3) eingesetzt. Die Enzyme werden in Form von Zelllysat eingesetzt.Der Ansatz wird auf 0,5 ml mit Wasser aufgefüllt. Die Reaktion findet für 24 h bei 40°C unter kontinuierlichem Schütteln (900 rpm) im offenen System statt, um genügend Sauerstoffversorgung für die NADH-Oxidase aus der Luft zu gewährleisten. Im offenen System bei 40°C verdampft Wasser. Es wird daher nach 6 h und 21 h mit Wasser auf 0,5 ml aufgefüllt. Es wird ein Umsatz der D-Glucose zu D-Fructose von ca. 98% erreicht.

### Beispiel 7

### Aufarbeitung und Analytik der Zucker

Der Ansatz wird für 10 min bei 65°C inkubiert um die Enzyme zu deaktivieren und anschließend zentrifugiert. Der Überstand wird dann über einen 0,2 µM PVDF Filter filtriert und mittels Ligand-Exchange-HPLC analysiert (Agilent Technologies Inc.). Aufgetrennt werden Zucker und Polyole dabei über eine Blei-Säule von Showa Denko K.K. (Shodex® Sugar SP0810) mit einem Fluss von 0,5 ml/min Wasser (VWR International GmbH, HPLC Grade) bei 80°C. Die Detektion erfolgt mittels Lichtbrechungsdetektor (RID, Agilent 1260 Infinity®, Agilent Technologies Inc.). Es wird ein Inlinefilter von Agilent Technologies Inc., sowie als Vorsäulen eine Anionen-Austauscher-Säule (Shodex® Axpak-WAG), eine Reversed-Phase Säule (Shodex® Asahipak® ODP-50 6E) und eine Zucker-Vorsäule (SUGAR SP-G) von Showa Denko K.K. verwendet.

### Beispiel 8

### Biokonversion von Cholsäure zu 3α,7β-Dihydroxy-12-oxo-5β-cholansäure durch 12α-Hydroxysteroiddehydrogenase, 7α-Hydroxysteroiddehydrogenase und 7β-Hydroxysteroiddehydrogenase unter der Verwendung eines Laktatdehydrogenase- und Alkoholdehydrogenase-abhängigen Kofaktor-Regenerierungssystems

Ein 0,5 ml-Ansatz enthält 25 mg Cholsäure, 12,5 U der rekombinanten 12α-Hydroxysteroiddehydrogenase aus *Eggertella lenta* oder *Lysinibacillus sphaericus*, 16 U der rekombinanten 7α-Hydroxysteroiddehydrogenase aus *Escherichia coli,* 6 U der rekombinanten 7β-Hydroxysteroiddehydrogenase aus *Ruminococcus torques* sowie 1 mM NAD⁺ und 1 mM NADPH. Zur Regenerierung von NAD⁺ werden 12,5 U der rekombinanten Laktatdehydrogenase aus *Oryctolagus cuniculus* (Muskel Isoform) und 200 mM Natrium-Pyruvat eingesetzt. Für die Regenerierung von NADPH werden 5 U der rekombinanten Alkoholdehydrogenase aus *Lactobacillus kefir* und anfänglich 2 % IPA (w/v) eingesetzt. Die Reaktion wird in einem wässrigen Kaliumphosphatpuffer (100 mM, pH 7.8) bei 25 °C unter kontinuierlichem Schütteln (850 rpm) durchgeführt. Es wird weiterhin ein offenes System verwendet, um die Verdampfung von Aceton zu ermöglichen und die Reaktion in Richtung 3α,7β-Dihydroxy-12-oxo-5β-cholansäure zu verschieben. Es werden 2% IPA nach 18 h, und 24 h nachdosiert. Nach 48 h sind 61 % der eingesetzten Cholsäure zu 3α,7α-dihydroxy-β-oxo-5β-cholansäure umgesetzt.

### Beispiel 9

### Biokonversion von Cholsäure zu 3α,7β-Dihydroxy-12-oxo-5β-cholansäure durch 12α-Hydroxysteroiddehydrogenase, 7a-Hydroxysteroiddehydrogenase und 7β-Hydroxysteroiddehydrogenase unter der Verwendung eines Laktatdehydrogenase-, NADH-Oxidase- und Alkoholdehydrogenase-abhängigen Kofaktor-Regenerierungssystems

Ein 0,5 ml-Ansatz enthält 25 mg Cholsäure, 12,5 U der rekombinanten 12α-Hydroxysteroiddehydrogenase aus *Eggertella lenta* oder *Lysinibacillus sphaericus,* 16 U der rekombinanten 7α-Hydroxysteroiddehydrogenase aus *Escherichia coli*, 6 U der rekombinanten 7β-Hydroxysteroiddehydrogenase aus *Ruminococcus torques* sowie 1 mM NAD⁺ und 1 mM NADPH. Zur Regenerierung von NAD+ werden 5 U der rekombinanten NADH-Oxidase aus *Leuconostoc mesenteroides* und 12,5 U der rekombinanten Laktatdehydrogenase aus *Oryctolagus cuniculus* (Muskel Isoform) und 200 mM Natrium-Pyruvat eingesetzt. Für die Regenerierung von NADPH werden 5 U der rekombinanten Alkoholdehydrogenase aus *Lactobacillus kefir* und anfänglich 2 % IPA (w/v) eingesetzt. Die Reaktion wird in einem wässrigen Kaliumphosphatpuffer (100 mM, pH 7.8) bei 25 °C unter kontinuierlichem Schütteln (850 rpm) durchgeführt. Es wird weiterhin ein offenes System verwendet, um die Verdampfung von Aceton zu ermöglichen und die Reaktion in Richtung 3α,7β-Dihydroxy-12-oxo-5β-cholansäure zu verschieben. Es werden 2 % IPA (w/v) nach 18 h, und 24 h nachdosiert. Nach 48 h sind 70% der eingesetzten Cholsäure zu 3α,7α-Dihydroxy-12-oxo-5β-cholansäure umgesetzt.

### Beispiel 10

### Epimerisierung von Chenodeoxycholsäure zu Ursodeoxycholsäure durch 7α-Hydroxysteroiddehydrogenase und 7β-Hydroxysteroiddehydrogenase unter der Verwendung eines Laktatdehydrogenase- und Alkoholdehydrogenase-abhängigen Kofaktor-Regenerierungssystems. Vorteil der Zugabe von Manganchlorid (MnCl₂)

Ein 0,5 ml-Ansatz enthält 50 mg Chenodeoxycholsäure, 12 U der rekombinanten 7α-Hydroxysteroiddehydrogenase aus *Escherichia coli,* 6 U der rekombinanten 7β-Hydroxysteroiddehydrogenase aus *Ruminococcus torques* sowie 0,5 mM NAD⁺ und 0,3 mM NADPH. Zur Regenerierung von NAD⁺ werden 6 U rekombinante Laktatdehydrogenase und 350 mM Natrium-Pyruvat eingesetzt. Für die Regenerierung von NADPH werden 6 U der rekombinanten Alkoholdehydrogenase aus *Lactobacillus kefir* und anfänglich 2,4% IPA (w/v) eingesetzt. Die Reaktion wird in einem wässrigen Kaliumphosphatpuffer (100 mM, pH = 7,8) mit 5 mM MnCl₂ bei 25°C und unter kontinuierlichem Schütteln (850 rpm) durchgeführt. Es wird weiterhin ein offenes System verwendet, um die Verdampfung von Aceton zu ermöglichen und die Reaktion in Richtung Ursodeoxycholsäure zu verschieben. Es werden 1,6% (w/v) IPA nach 6 h, 2,4% (w/v) IPA nach 16 h und 3,9% (w/v) IPA nach 24 h nachdosiert. Nach 36 h werden 200 µl 2-Pentanol sowie 3 %(w/v) IPA zugegeben und nach 48 h werden 100 µl 2-Pentanol und 4 % (w/v) IPA nachdosiert. Nach 64 h beträgt der Anteil von Ursodeoxycholsäure an allen Gallensäuren in der Reaktionsmischung >99%. Es beträgt insbesondere der Anteil von Chenodeoxycholsäure ca. 0,3 %. Bei einem Kontrollansatz ohne Zugabe von MnCl₂ liegt der Anteil von Chenodeoxycholsäure bei ca. 2 % und der Anteil von Ursodeoxycholsäure bei ca. 97,5 % (jeweils Mittelwerte aus 5 Experimenten).

### Beispiel 11

### Epimerisierung von Chenodeoxycholsäure zu Ursodeoxycholsäure durch 7α-Hydroxysteroiddehydrogenase und 7β-Hydroxysteroiddehydrogenase unter der Verwendung eines Alkoholdehydrogenase-abhängigen Kofaktor-Regenerierungssystems sowie eines kombinierten Laktatdehydrogenase- und NADH-Oxidase-abhängigen Kofaktor-Regenerierungssystems

Ein 0,5 ml-Ansatz enthält 50 mg Chenodeoxycholsäure, 12 U der rekombinanten 7α-Hydroxysteroiddehydrogenase aus *Escherichia coli,* 6 U der rekombinanten 7β-Hydroxysteroiddehydrogenase aus *Ruminococcus torques* sowie 0,5 mM NAD⁺ und 0,3 mM NADPH. Zur Regenerierung von NAD⁺ werden 6 U rekombinante Laktatdehydrogenase und 350 mM Natrium-Pyruvat eingesetzt. Zur Regenerierung von NAD⁺ werden zusätzlich 9 U der rekombinanten NADH Oxidase aus *Leuconostoc mesenteroides* sowie 6 U der rekombinanten NADH Oxidase aus *Clostridium aminovalericum* eingesetzt. Für die Regenerierung von NADPH werden 6 U der rekombinanten Alkoholdehydrogenase aus *Lactobacillus kefir* und anfänglich 2,4% IPA (w/v) eingesetzt. Die Reaktion wird in einem wässrigen Kaliumphosphatpuffer (100 mM, pH = 7,8) bei 25°C und unter kontinuierlichem Schütteln (850 rpm) durchgeführt. Es wird weiterhin ein offenes System verwendet, um die Verdampfung von Aceton zu ermöglichen und die Reaktion in Richtung Ursodeoxycholsäure zu verschieben. Es werden 1,6% (w/v) IPA nach 6 h, 2,4% (w/v) IPA nach 16 h und 3,9% (w/v) IPA nach 24 h nachdosiert. Nach 36 h werden 200 µl 2-Pentanol sowie 3 % (w/v) IPA zugegeben und nach 48 h werden 100 µl 2-Pentanol und 4% (w/v) IPA nachdosiert. Nach 64 h beträgt der Anteil von Ursodeoxycholsäure an allen Gallensäuren in der Reaktionsmischung >99%. Es beträgt insbesondere der Anteil von Chenodeoxycholsäure ca. 0,2 %. Bei einem Kontrollansatz ohne Zugabe von NADH-Oxidase liegt der Anteil von Chenodeoxycholsäure bei ca. 2 % und der Anteil von Ursodeoxycholsäure bei ca. 97,5 % (gleicher Kontrollansatz wie für Beispiel 11; jeweils Mittelwerte aus 5 Experimenten).

### Beispiel 12

### Epimerisierung von Chenodeoxycholsäure zu Ursodeoxycholsäure durch 7α-Hydroxysteroiddehydrogenase und 7β-Hydroxysteroiddehydrogenase unter der Verwendung eines Alkoholdehydrogenase-abhängigen Kofaktor-Regenerierungssystems sowie eines kombinierten Laktatdehydrogenase- und NADH-Oxidase-abhängigen Kofaktor-Regenerierungssystems. Additiver Effekt von 2-Pentanol und 2-Propanol

Ein 50 ml-Ansatz enthält 5 g Chenodeoxycholsäure, 24 U/ml der rekombinanten 7α-Hydroxysteroiddehydrogenase aus *Escherichia coli,* 12 U/ml der rekombinanten 7β-Hydroxysteroiddehydrogenase aus *Ruminococcus torques* sowie 0,5 mM NAD⁺ und 0,3 mM NADPH. Zur Regenerierung von NAD⁺ werden 12 U/ml rekombinante Laktatdehydrogenase und 350 mM Natrium-Pyruvat eingesetzt. Zur Regenerierung von NAD⁺ werden zusätzlich 18 U/ml der rekombinanten NADH Oxidase aus *Leuconostoc mesenteroides* sowie 12 U/ml der rekombinanten NADH Oxidase aus *Clostridium aminovalericum* eingesetzt. Für die Regenerierung von NADPH werden 12 U/ml der rekombinanten Alkoholdehydrogenase aus *Lactobacillus kefir* und anfänglich 1,5 % IPA (w/v) eingesetzt. Die Reaktion wird in einem wässrigen Kaliumphosphatpuffer (100 mM, pH = 7,8) mit 5 mM MnCl₂ bei 25°C durchgeführt. In einem 3-Halskolben wird mit einem KPG-Rührer bei ca. 100 rpm gerührt. Eine Entfernung des bei der Reaktion entstehenden Acetons wird durch einen Luftstrom von ca. 400-600 ml/min durch das Reaktionsgefäß gewährleistet. Da ebenfalls 2-Propanol verdampft, sind Nachdosierungen notwendig. Diese betragen im Beispiel 0,75 ml (1,5 h), 0,75 ml (3 h), 0,5 ml (4 h), 0,75 ml (6 h), 0,75 ml (8 h), 0,5 ml (11 h), 0,5 ml (14 h), 0,5 ml (17 h), 0,5 ml (21 h), 1 ml (23 h), 2,5 ml (25 h), 4 ml (29 h). Nach ca. 30 h werden 20 ml 2-Pentanol sowie 2 ml 2-Propanol zugegeben. Nach 46 h Gesamt-Reaktionszeit beträgt der Anteil von 7-Ketolithocholsäure ca. 1 % (bezogen auf die Summe von Chenodeoxycholsäure, Ursodeoxycholsäure und 7-Ketolithocholsäure). Es wird nun weiter 2-Propanol zugegeben: 3 ml (46 h), 4 ml (52 h), 4 ml (54 h) sowie zusätzlich 10 ml 2-Pentanol. Nach 72 h Gesamt-Reaktionszeit kann der Anteil von 7-Ketolithocholsäure auf weniger als 0,2 % gesenkt werden. Der Anteil von Ursodeoxycholsäure beträgt >99 %.

### Beispiel 13

### Aufarbeitung und Analytik der Gallensäuren

Nach Beendigung von Reaktionen, wie sie in den Beispielen 8 bis 12 beschrieben sind, können die in den Proben enthaltenen Gallensäuren mittels einer Methode wie sie in Beispiel 4 beschrieben ist analysiert werden.

## Patentansprüche

1. Verfahren zur enzymatischen Regenerierung der Redoxkofaktoren NAD⁺/NADH und NADP⁺/NADPH in einer Ein-Topf-Reaktion, wobei als Resultat mindestens zweier weiterer im selben Reaktionsansatz ablaufender enzymatisch katalysierter Redoxreaktionen (Produktbildungsreaktionen) der Redoxkofaktor NAD⁺/NADH in seiner reduzierten Form als NADH anfällt und der Redoxkofaktor NADP⁺/NADPH in seiner oxidierten Form als NADP⁺, **dadurch gekennzeichnet, dass**
a) bei der Regenerierungsreaktion, die NADH wieder in NAD⁺ überführt, Sauerstoff mittels einer NADH Oxidase oder Pyruvat mittels einer Laktatdehydrogenase reduziert wird, und
b) bei der Regenerierungsreaktion, die NADP⁺ wieder in NADPH überführt, 2-Propanol mittels einer Alkoholdehydrogenase oder Malat mittels einer Malatdehydrogenase oxidiert wird.

2. Verfahren nach Anspruch 1, **dadurch gekennzeichnet, dass** Oxidationsreaktion(en) und Reduktionsreaktion(en) am selben Substrat (Molekülgrundgerüst) stattfinden.

3. Verfahren nach Anspruch 1 oder 2, **dadurch gekennzeichnet, dass** Oxidationsreaktion(en) und Reduktionsreaktion(en) zeitlich parallel ablaufen.

4. Verfahren nach einem der Ansprüche 1 bis 3, **dadurch gekennzeichnet, dass** bei der Regenerierungsreaktion, die NADP⁺ in NADPH überführt, mittels einer Alkoholdehydrogenase 2-Propanol zu Aceton oxidiert wird.

5. Verfahren nach einem der Ansprüche 1 bis 3, **dadurch gekennzeichnet, dass** bei der Regenerierungsreaktion, die NADH in NAD⁺ überführt, mittels einer Laktatdehydrogenase Pyruvat zu Laktat reduziert wird.

6. Verfahren nach Anspruch 5, **dadurch gekennzeichnet, dass** bei der Regenerierungsreaktion, die NADP⁺ in NADPH überführt, mittels einer Malatdehydrogenase Malat zu Pyruvat und CO₂ oxidiert wird.

7. Verfahren nach einem der Ansprüche 1 bis 5, **dadurch gekennzeichnet, dass** es eingesetzt wird zur Durchführung von jeweils mindestens einer Oxidationsreaktion und mindestens einer Reduktionsreaktion im selben Reaktionsansatz an Verbindungen der allgemeinen Formel 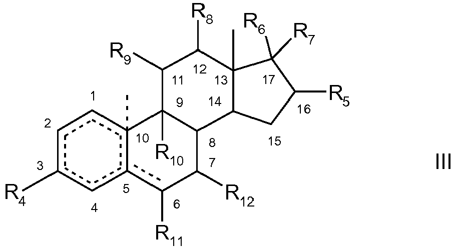 worin
R₄ Wasserstoff, eine Methylgruppe, eine Hydroxygruppe oder eine Oxogruppe,
R₅ Wasserstoff, eine Hydroxygruppe, eine Oxogruppe oder eine Methylgruppe,
R₆ Wasserstoff oder eine Hydroxygruppe,
R₇ Wasserstoff, -COR₁₃, worin R₁₃ eine unsubstituierte oder mit einer Hydroxygruppe substituierte C₁-C₄-Alkylgruppe ist, oder eine substituierte, insbesondere mit einer Hydroxygruppe, oder unsubstituierte C₁-C₄ Carboxyalkylgruppe,
oder R₆ und R₇ zusammen eine Oxogruppe bedeuten,
R₈ Wasserstoff, eine Methylgruppe, eine Hydroxygruppe oder eine Oxogruppe,
R₉ Wasserstoff, eine Methylgruppe, eine Hydroxygruppe oder eine Oxogruppe,
R₁₀ Wasserstoff, eine Methylgruppe oder Halogen,
R₁₁ Wasserstoff, eine Methylgruppe, eine Hydroxygruppe, eine Oxogruppe oder Halogen, und
R₁₂ Wasserstoff, eine Hydroxygruppe, eine Oxogruppe oder eine Methylgruppe bedeuten,
wobei das Strukturelement 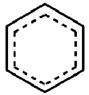 einen Benzolring oder einen Ring mit 6 Kohlenstoffatomen und 0, 1 oder 2 C-C-Doppelbindungen bedeutet; insbesondere wobei das Substrat/die Substrate für die an der Produktbildung beteiligte(n) Reduktionsreaktion(en) in einer Konzentration von <5% (w/v) im Reaktionsansatz vorliegt/vorliegen.

8. Verfahren nach Anspruch 7, **dadurch gekennzeichnet, dass** es eingesetzt wird für die Umwandlung von Dehydroepiandrosteron (DHEA) der Formel 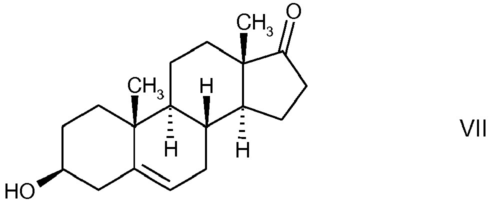 in Testosteron der Formel

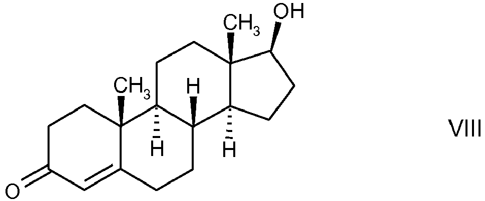

9. Verfahren nach Anspruch 7, **dadurch gekennzeichnet, dass** es eingesetzt wird zur enzymatischen Epimerisierung von 3α,7α-Dihydroxy-5β-cholansäure (Chenodeoxycholsäure) der Formel 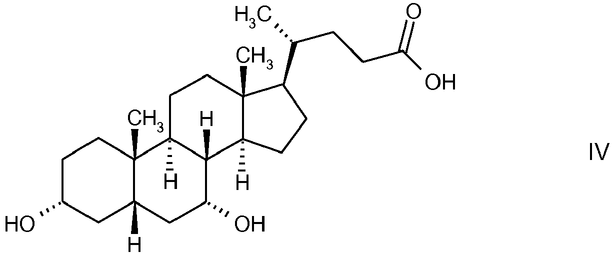 durch Oxidation zu Ketolithocholsäure der Formel 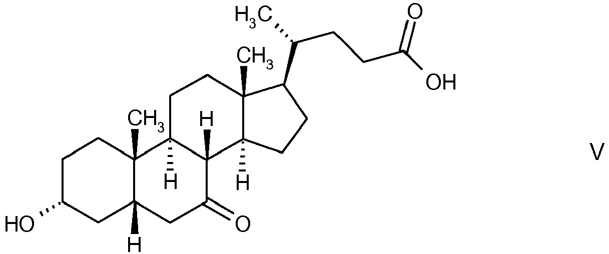 und nachfolgende Reduktion zur stereoisomeren Hydroxyverbindung 3α,7β-Dihydroxy-5β-cholansäure (Ursodeoxycholsäure) der Formel 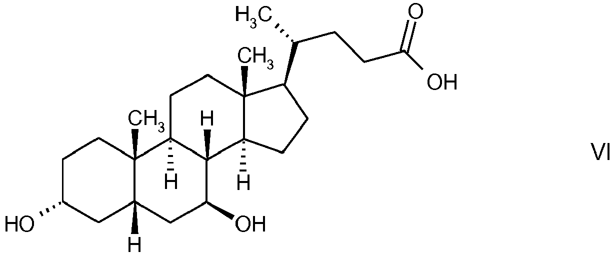 mittels zweier entgegengesetzt stereospezifischer Hydroxysteroiddehydrogenasen, wobei gegebenenfalls die Oxidationsreaktion durch eine 7α-Hydroxysteroiddehydrogenase aus *E. coli* und/oder die Reduktionsreaktion durch eine 7β-Hydroxysteroiddehydrogenase aus *Ruminococcus torques* katalysiert wird.

10. Verfahren nach Anspruch 7, **dadurch gekennzeichnet, dass** es eingesetzt wird zur enzymatischen Epimerisierung von 3α,7α,12α-Trihydroxy-5β-cholansäure (Cholsäure) der Formel 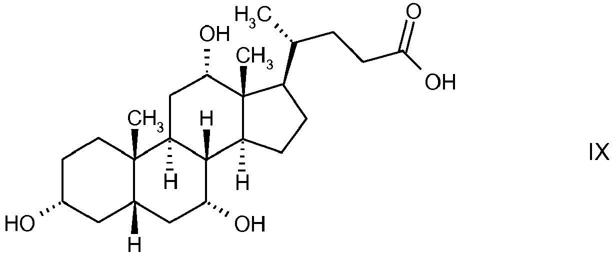 entweder
A) durch Oxidation zu 3α,7α-Dihydroxy-12-oxo-5β-cholansäure (12-oxo-CDC) der Formel 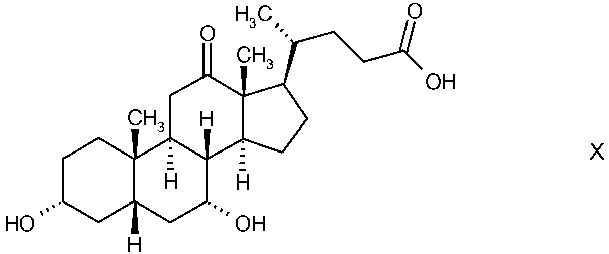 die weiter umgesetzt wird zur 3α-Hydroxy-7,12-dioxo-5β-cholansäure (12oxo-KLC) der Formel 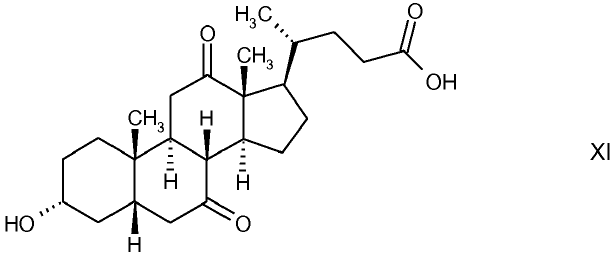 und nachfolgende Reduktion zur stereoisomeren Hydroxyverbindung 3α,7β-Dihydroxy-12-oxo-5β-cholansäure (12-Keto-Ursodeoxycholsäure) der Formel 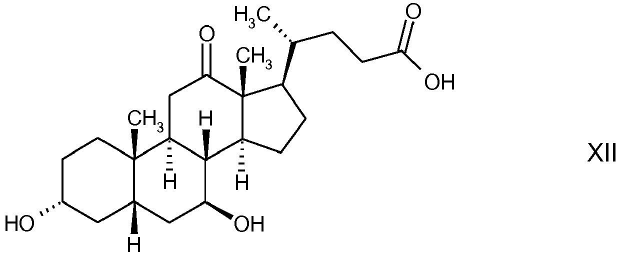 oder
B) durch Oxidation zu 3α,12α-Dihydroxy-7-oxo-5β-cholansäure der Formel 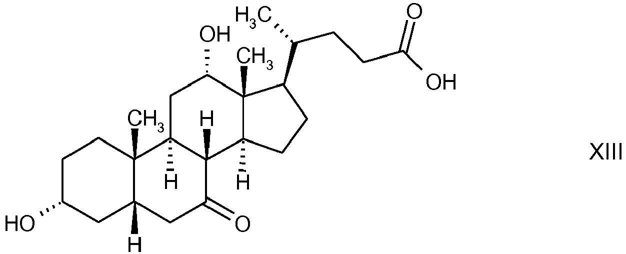 gefolgt von enzymatischer Oxidation zur 3α-Hydroxy-7,12-dioxo-5β-cholansäure (12oxo-KLC) der Formel 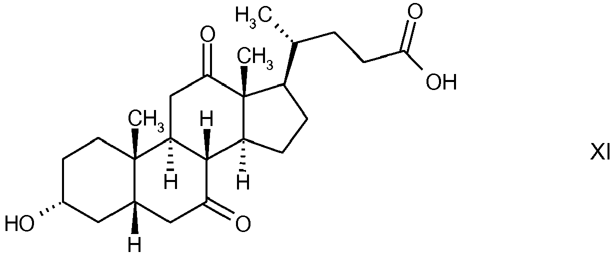 und nachfolgende Reduktion zur stereoisomeren Hydroxyverbindung 3α,7β-Dihydroxy-12-oxo-5β-cholansäure (12-Keto-Ursodeoxycholsäure) der Formel XII, oder
C) durch Oxidation zu 3α,12α-Dihydroxy-7-oxo-5β-cholansäure der Formel XIII, gefolgt von enzymatischer Reduktion zur 3α,7β,12α-Triydroxy-5β-cholansäure der Formel 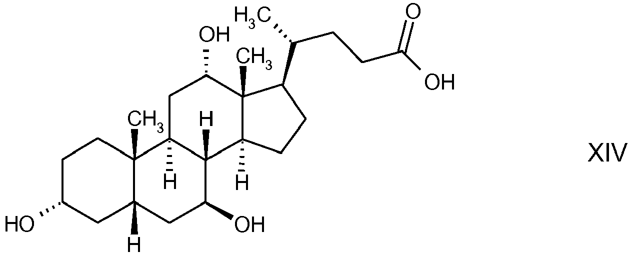 und nachfolgende Oxidation zur stereoisomeren Hydroxyverbindung 3α,7β-Dihydroxy-12-oxo-5β-cholansäure (12-Keto-Ursodeoxycholsäure) der Formel XII; mittels dreier stereospezifischer Hydroxysteroiddehydrogenasen; wobei 2 der drei stereospezifischer Hydroxysteroiddehydrogenasen entgegengesetzt spezifisch sind.

11. Verfahren nach einem der Ansprüche 1 bis 6, **dadurch gekennzeichnet, dass** es eingesetzt wird zur Isomerisierung von C₅- oder C₆-Zuckern, insbesondere zur Isomerisierung von Glukose durch Reduktion zu Sorbitol und nachfolgende Oxidation zu Fruktose.

12. Verfahren nach einem der Ansprüche 1 bis 11, **dadurch gekennzeichnet, dass** das Substrat/die Substrate für die an der Produktbildung beteiligte(n) Oxidationsreaktion(en) in einer Konzentration von 5% (w/v) und mehr, insbesondere von 7% (w/v) und mehr, insbesondere von 9% (w/v) und mehr im Reaktionsansatz vorliegt/vorliegen.

## Claims

1. Process for the enzymatic regeneration of the redox cofactors NAD⁺/NADH and NADP⁺/NADPH in a one-pot reaction, wherein, as a result of at least two further enzymatically catalyzed redox reactions proceeding in the same reaction batch (product-forming reactions), the redox cofactor NAD⁺/NADH accumulates in its reduced form as NADH and the redox cofactor NADP⁺/NADPH accumulates in its oxidized form as NADP⁺, **characterized in that**
a) in the regeneration reaction which reconverts NADH into NAD⁺, oxygen is reduced by means of an NADH oxidase, or pyruvate by means of a lactate dehydrogenase, and
b) in the regeneration reaction which reconverts NADP⁺ into NADPH, 2-propanol is oxidized by means of an alcohol dehydrogenase, or malate by means of a malate dehydrogenase.

2. Process according to claim 1, **characterized in that** oxidation reaction(s) and reduction reaction(s) take place on the same substrate (molecular backbone).

3. Process according to claim 1 or 2, **characterized in that** oxidation reaction(s) and reduction reaction(s) proceed chronologically parallel.

4. Process according to any of claims 1 to 3, **characterized in that**, in the regeneration reaction which reconverts NADP⁺ into NADPH, 2-propanol is oxidized to acetone by means of an alcohol dehydrogenase.

5. Process according to any of claims 1 to 3, **characterized in that**, in the regeneration reaction which reconverts NADH into NAD⁺, pyruvate is reduced to lactate by means of a lactate dehydrogenase.

6. Process according to claim 5, **characterized in that**, in the regeneration reaction which reconverts NADP⁺ into NADPH, malate is oxidized to pyruvate and CO₂ by means of a malate dehydrogenase.

7. Process according to any of claims 1 to 5, **characterized in that** it is used for performing at least one oxidation reaction and at least one reduction reaction, respectively, in the same reaction batch on compounds of general formula 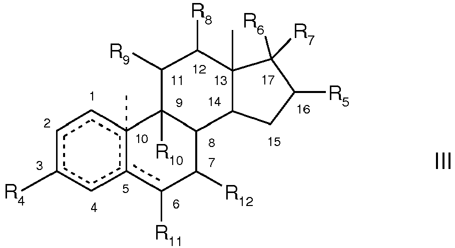 wherein
R₄ denotes hydrogen, a methyl group, a hydroxy group or an oxo group,
R₅ denotes hydrogen, a hydroxy group, an oxo group or a methyl group,
R₆ denotes hydrogen or a hydroxy group,
R₇ denotes hydrogen, -COR₁₃, wherein R₁₃ is a C₁-C₄ alkyl group which is unsubstituted or substituted with a hydroxy group, or a C₁-C₄ carboxy alkyl group which is substituted, in particular with a hydroxy group, or unsubstituted,
or R₆ and R₇ together denote an oxo group,
R₈ denotes hydrogen, a methyl group, a hydroxy group or an oxo group,
R₉ denotes hydrogen, a methyl group, a hydroxy group or an oxo group,
R₁₀ denotes hydrogen, a methyl group or halogen,
R₁₁ denotes hydrogen, a methyl group, a hydroxy group, an oxo group or halogen, and
R₁₂ denotes hydrogen, a hydroxy group, an oxo group or a methyl group,
wherein the structural element 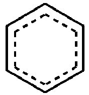
denotes a benzene ring or a ring comprising 6 carbon atoms and 0, 1 or 2 C-C-double bonds; in particular wherein the substrate(s) is/are provided at a concentration of <5% (w/v) in the reaction batch for the reduction reaction(s) involved in the formation of the product.

8. Process according to claim 7, **characterized in that** it is used for the conversion of dehydroepiandrosterone (DHEA) of formula 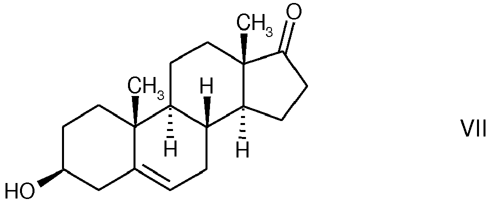 into testosterone of formula

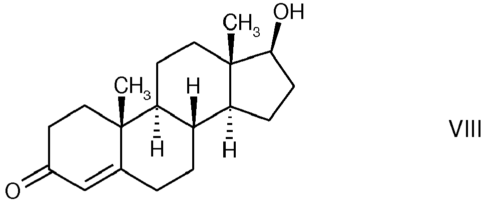

9. Process according to claim 7, **characterized in that** it is used for the enzymatic epimerization of 3α,7α-dihydroxy-5β-cholanic acid (chenodeoxycholic acid) of formula 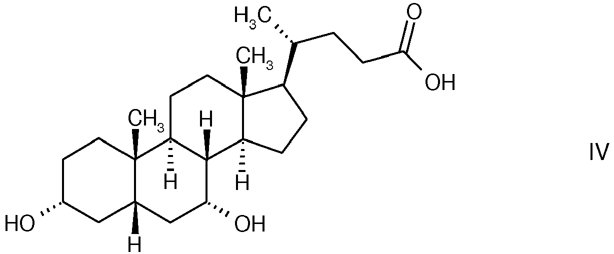 into ketolithocholic acid of formula 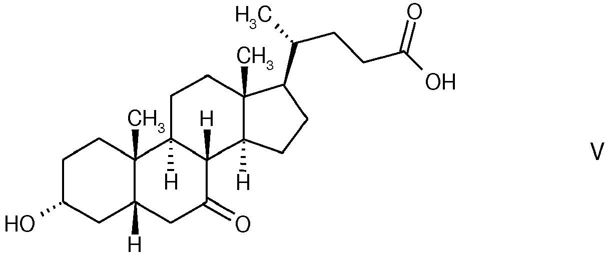 by oxidation,
and into the stereoisomeric hydroxy compound 3α,7β-dihydroxy-5β-cholanic acid (ursodeoxycholic acid) of formula 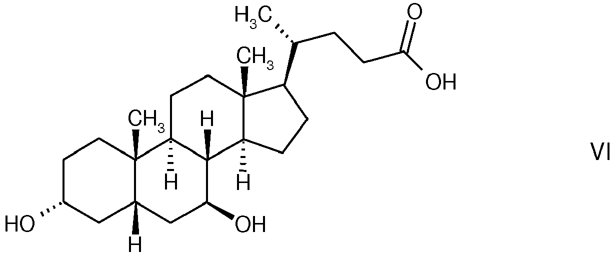 by subsequent reduction,
using two opposite stereospecific hydroxysteroid dehydrogenases, wherein, optionally, the oxidation reaction is catalyzed by a 7α-hydroxysteroid dehydrogenase from *E. coli,* and/or the reduction reaction is catalyzed by a 7β-hydroxysteroid dehydrogenase from *Ruminococcus torques.*

10. Process according to claim 7, **characterized in that** it is used for the enzymatic epimerization of 3α,7α,12α-trihydroxy-5β-cholanic acid (cholic acid) of formula 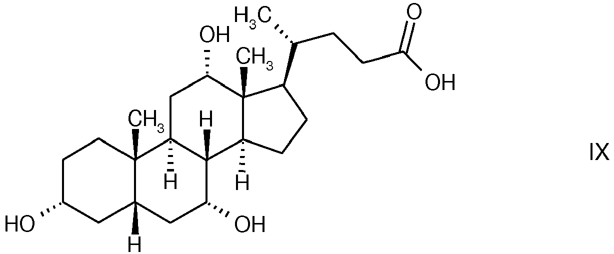 either
A) via oxidation to obtain 3α,7α-dihydroxy-12-oxo-5β-cholanic acid (12-oxo-CDC) of formula 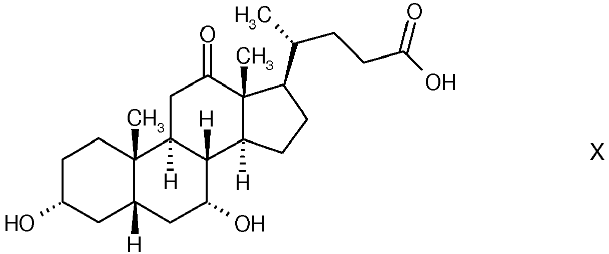 which is further reacted to obtain 3α-hydroxy-7,12-dioxo-5β-cholanic acid (12oxo-KLC) of formula 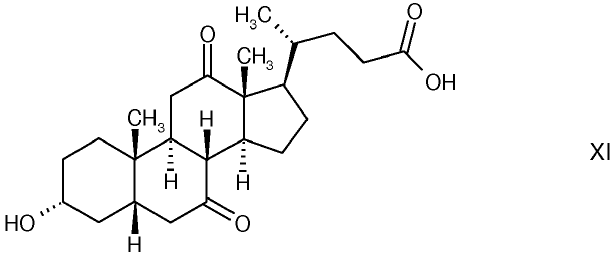 and subsequent reduction to the stereoisomeric hydroxy compound 3α,7β-dihydroxy-12-oxo-5β-cholanic acid (12-keto-ursodeoxycholic acid) of formula 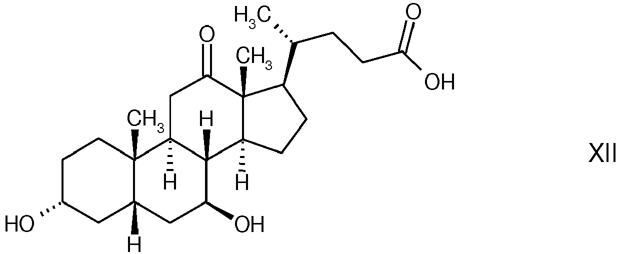 or
B) via oxidation to obtain 3α,12α-dihydroxy-7-oxo-5β-cholanic acid of formula 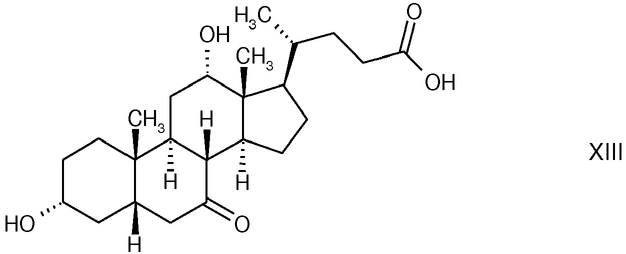 followed by enzymatic oxidation to obtain 3α-hydroxy-7,12-dioxo-5β-cholanic acid (12oxo-KLC) of formula 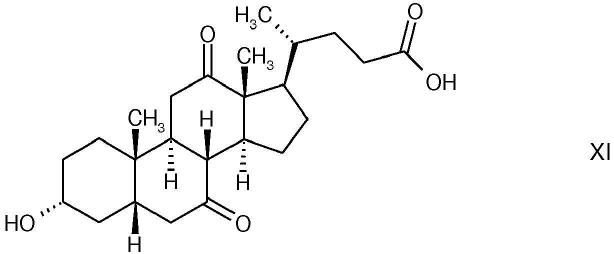 and subsequent reduction to obtain the stereoisomeric hydroxy compound 3α,7β-dihydroxy-12-oxo-5β-cholanic acid (12-keto-ursodeoxycholic acid) of formula XII, or
C) via oxidation to obtain 3α,12α-dihydroxy-7-oxo-5β-cholanic acid of formula XIII, followed by enzymatic reduction to obtain 3α,7β,12α-triydroxy-5β-cholanic acid of formula 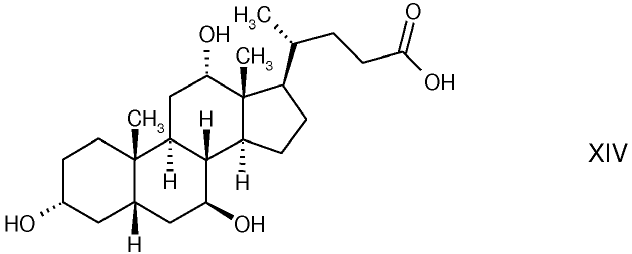 and subsequent oxidation to obtain the stereoisomeric hydroxy compound 3α,7β-dihydroxy-12-oxo-5β-cholanic acid (12-keto-ursodeoxycholic acid) of formula XII;
using three stereospecific hydroxysteroid dehydrogenases; with 2 of the three stereospecific hydroxysteroid dehydrogenases having opposite specifity.

11. Process according to any of claims 1 to 6, **characterized in that** it is used for the isomerization of C₅- or C₆-sugars, in particular for the isomerization of glucose via reduction to sorbitol and subsequent oxidation to fructose.

12. Process according to any of claims 1 to 11, **characterized in that** the substrate(s) for the oxidation reaction(s) involved in the formation of a product is/are provided in the reaction batch at a concentration of 5% (w/v) and more, in particular 7% (w/v) and more, in particular 9% (w/v) and more.

## Revendications

1. Procédé de régénération enzymatique de cofacteurs redox NAD⁺/NADH et NADP⁺/NADPH dans une réaction en monoréacteur, le cofacteur redox NAD⁺/NADH étant obtenu dans sa forme réduite en tant que NADH et le cofacteur NADP⁺/NADPH étant obtenu dans sa forme oxydée en tant que NADP⁺ en résultat d'au moins deux autres réactions redox (réactions de formation de produits) à catalyse enzymatique se déroulant lors du même processus de réaction, **caractérisé en ce que**
a) lors de la réaction de régénération qui reconvertit le NADH en NAD⁺, de l'oxygène est réduit au moyen d'une NADH oxydase ou d'un pyruvate au moyen d'une lactate déshydrogénase, et
b) lors de la réaction de régénération, qui reconvertit le NADP⁺ en NADPH, du 2-propanol est oxydé au moyen d'une alcool déshydrogénase ou du malate au moyen d'une malate déshydrogénase.

2. Procédé selon la revendication 1, caractérisé selon la revendication 1, **caractérisé en ce que** la (les) réaction(s) d'oxydation et la (les) réaction(s) de réduction ont lieu sur le même substrat (squelette moléculaire).

3. Procédé selon la revendication 1 ou la revendication 2, **caractérisé en ce qu'**une (des) réaction(s) d'oxydation et une (des) réaction(s) de réduction se déroulent en parallèle dans le temps.

4. Procédé selon l'une quelconque des revendications 1 à 3, **caractérisé en ce que**, lors de la réaction de régénération, qui convertit le NADP⁺ en NADPH, du 2-propanol est oxydé en acétone au moyen d'une alcool déshydrogénase.

5. Procédé selon l'une quelconque des revendications 1 à 3, **caractérisé en ce que**, lors de la réaction de régénération, qui convertit le NADH en NAD⁺, du pyruvate est réduit en lactate au moyen d'une lactate déshydrogénase.

6. Procédé selon la revendication 5, **caractérisé en ce que**, lors de la réaction de régénération, qui convertit le NADP⁺ en NADPH, du malate est oxydé en pyruvate et en CO₂ au moyen d'une malate déshydrogénase.

7. Procédé selon l'une quelconque des revendications 1 à 5, **caractérisé en ce qu'**il est utilisé pour procéder à au moins une réaction d'oxydation et au moins à une réaction de réduction dans le même processus de réaction sur des composés de formule générale 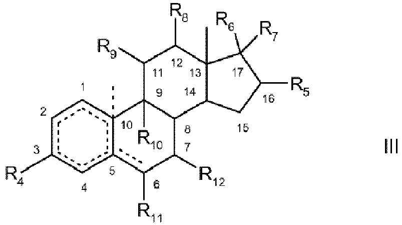 dans laquelle
R₄ représente de l'hydrogène, un radical méthyle, un radical hydroxyle ou un radical oxo,
R₅ de l'hydrogène, un radical hydroxyle, un radical oxo ou un radical méthyle,
R₆ de l'hydrogène ou un radical hydroxyle,
R₇ de l'hydrogène, un groupement -COR₁₃, dans lequel R₁₃ est un radical alkyle en C₁ à C₄ non substitué ou substitué avec un radical hydroxyle, ou bien un radical carboxyalkyle en C₁ à C₄ substitué, en particulier avec un radical hydroxyle ou non substitué,
ou R₆ et R₇ représentent ensemble un radical oxo,
R₈ représente de l'hydrogène, un radical méthyle, un radical hydroxyle ou un radical oxo,
R₉ de l'hydrogène, un radical méthyle, un radical hydroxyle ou un radical oxo,
R₁₀ de l'hydrogène, un radical méthyle ou un groupement halogène,
R₁₁ de l'hydrogène, un radical méthyle, un radical hydroxyle, un radical oxo ou un groupement halogène ou
R₁₂ de l'hydrogène, un radical hydroxyle, un radical oxo ou un radical méthyle,
l'élément structurel 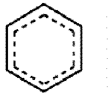 représentant un cycle benzène ou un cycle portant 6 atomes de carbone et 0,1 ou 2 doubles liaisons C-C ; en particulier, le substrat/les substrats destinés à la (aux) réaction(s) de réduction impliquée(s) dans la formation de produits se présentant dans une concentration < 5 % (w/v) dans le processus de réaction.

8. Procédé selon la revendication 7, **caractérisé en ce qu'**il est utilisé pour la transformation de déshydroépiandrostérone (DHEA) de formule 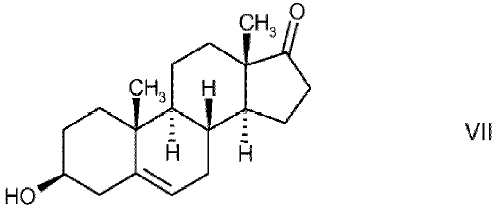 dans de la testostérone de formule

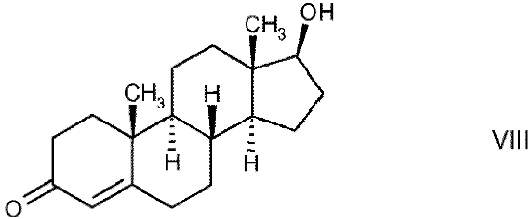

9. Procédé selon la revendication 7, **caractérisé en ce qu'**il est utilisé pour l'épimérisation enzymatique d'acide 3α,7α-dihydroxy-5β-cholanique (acide chénodésoxycholique) de formule 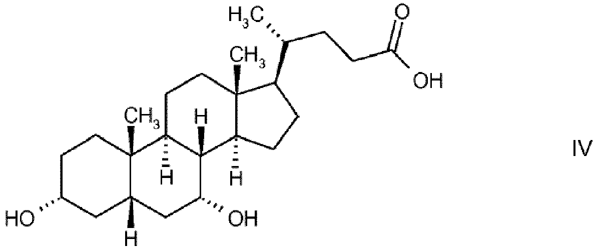 par oxydation en acide cétolithocholique de formule 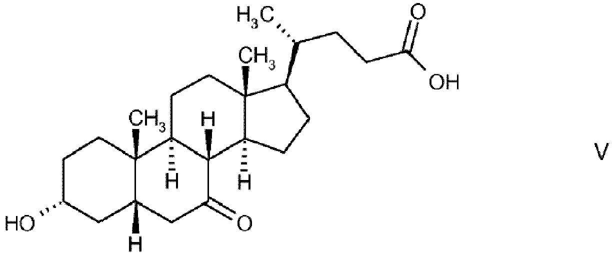 et réduction subséquente pour le composé hydroxyle stéréoisomère acide 3α,7β-dihydroxy-5β-cholanique (acide ursodésoxycholique) de formule 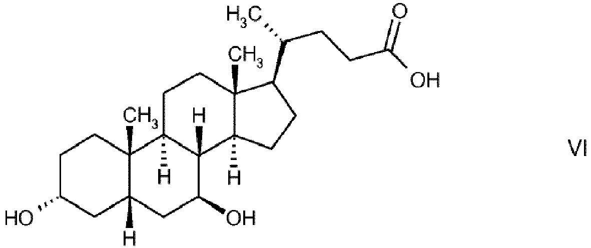 au moyen de deux hydroxystéroïde déshydrogénases stéréospécifiques opposées, la réaction d'oxydation étant catalysée, le cas échéant, par une 7α-hydroxystéroïde déshydrogénase à partir de E. *coli* et/ou la réaction de réduction par une 7β-hydroxystéroide déshydrogénase à partir de *Rumincococcus torques.*

10. Procédé selon la revendication 7, **caractérisé en ce qu'**il est utilisé pour l'épimérisation enzymatique d'acide 3α,7α,12α-trihydroxy-5β-cholanique (acide cholique) de formule 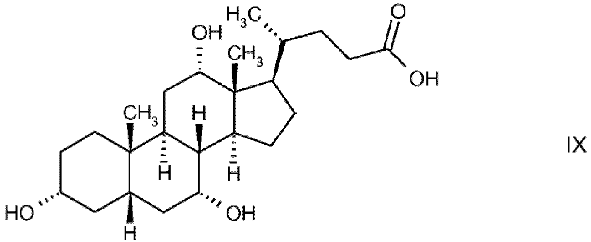 soit
A) par oxydation en acide 3α,7α-dihydroxy-12-oxo-5β-cholanique (12-oxo-CDC) de formule 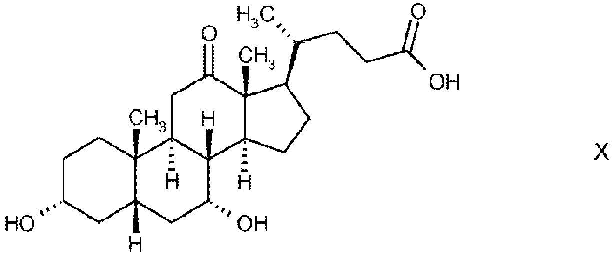 transformé ensuite en acide 3α-hydroxy-7,12-dioxo-5β-cholanique (12oxo-KLC) de formule 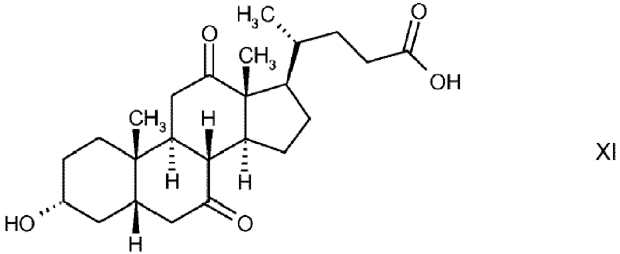 et réduction subséquente en composé hydroxyle stéréoisomère acide 3α,7β-dihydroxy-12-oxo-5β-cholanique (acide 12-céto-ursodéoxycholique) de formule 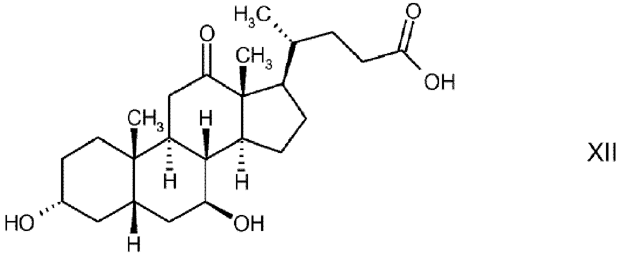 soit
B) par oxydation en acide 3α,12α-dihydroxy-7-oxo-5β-cholanique de formule 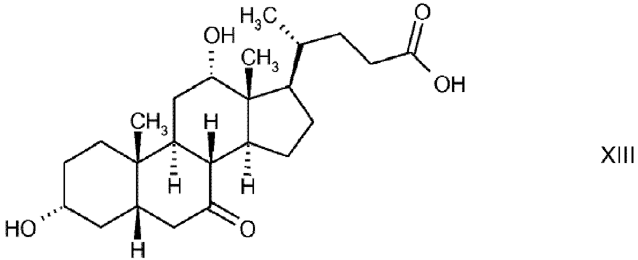 suivie d'une oxydation enzymatique en acide 3α-hydroxy-7,12-dioxo-5β-cholanique (12oxo-KLC) de formule 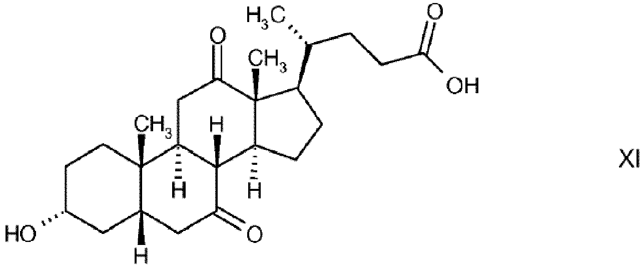 et réduction subséquente en composé hydroxyle stéréoisomère 3α,7β-dihydroxy-12-oxo-5β-cholanique (acide 12-céto-ursodésoxycholique) de formule XII,
soit
C) par oxydation en acide 3α,12α-dihydroxy-7-oxo-5-β-cholanique de formule XIII, suivie d'une oxydation enzymatique en acide 3α,7β,12α-trihydroxy-5β-cholanique de formule 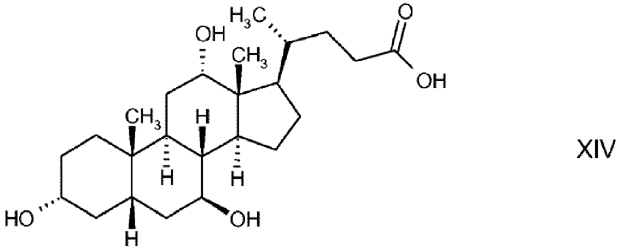
et oxydation subséquente en composé hydroxyle stéréoisomère 3α,7β-dihydroxy-12-oxo-5β-cholanique (acide 12-céto-ursodésocycholique) de formule XII ; au moyen de trois hydroxystéroïde déshydrogénases stéréospécifiques ; 2 des trois hydroxystéroïde déshydrogénases stéréospécifiques étant contrairement spécifiques.

11. Procédé selon l'une quelconque des revendications 1 à 6, **caractérisé en ce qu'**il est utilisé pour l'isomérisation de sucres en C₅ ou C₆, en particulier pour l'isomérisation de glucose par réduction en sorbitol et oxydation subséquente en fructose.

12. Procédé selon l'une quelconque des revendications 1 à 11, **caractérisé en ce que** le substrat/les substrats se présente (nt), pour la (les) réaction(s) d'oxydation impliquée(s) dans la formation de produits, dans une concentration de 5 % (w/v) et plus, en particulier de 7 % (w/v) et plus, en particulier de 9 % (w/v) et plus dans le processus de réaction.
